# EUROPÄISCHE PATENTSCHRIFT

(11) **EP 2 571 923 B1**
(45) Veröffentlichungstag und Bekanntmachung des Hinweises auf die Patenterteilung: **17.12.2014**
(21) Anmeldenummer: 11720463.6
(22) Anmeldetag: 18.05.2011
(51) Int. Cl.: C07C 43/23, C08G 65/22, C08G 65/26

(54) **DERIVATE VON TRIS(2-HYDROXYPHENYL)METHAN, DEREN HERSTELLUNG UND VERWENDUNG**
DERIVATIVES OF TRIS(2-HYDROXYPHENYL)METHANE, AND PREPARATION AND USE THEREOF
DÉRIVÉS DE TRIS(2-HYDROXYPHÉNYL)MÉTHANE, LEUR PRODUCTION ET LEUR UTILISATION

(30) Priorität: 20.05.2010 EP 10163371
(43) Veröffentlichungstag der Anmeldung: 27.03.2013
(73) Patentinhaber: BASF SE, 67056 Ludwigshafen (DE)
(72) Erfinder: REINOSO GARCIA, Marta, 69221 Dossenheim (DE); OETTER, Günter, 67227 Frankenthal (DE); KURKAL-SIEBERT, Vandana, 69117 Heidelberg (DE); HEINZ, Björn, 68159 Mannheim (DE); BITTNER, Christian, 64625 Bensheim (DE); HANSCH, Markus, 67346 Speyer (DE); RAETHER, Roman Benedikt, 67346 Speyer (DE)
(86) Internationale Anmeldenummer: PCT/EP2011/058011
(87) Internationale Veröffentlichungsnummer: WO 2011/144643

(56) Entgegenhaltungen:
- EP-A1- 0 597 806
- SHVETS V ET AL: "Stabilization of Finely Dispersed Suspensions of Water-Insoluble Organic Dyes by Nonionic Surfactants", JOURNAL OF APPLIED CHEMISTRY OF USSR, CONSULTANTS BUREAU, NEW YORK, NY, US, Bd. 58, Nr. 6, 1. Januar 1985 (1985-01-01) , Seiten 1220-1224, XP009116575, ISSN: 0021-888X
- LEMYRE J.-L ET AL.: "Characterization of a reverse micellar system by 1H NMR", LANGMUIR, Bd. 26, 25. Januar 2010 (2010-01-25), Seiten 6250-6255, XP002650894,

## Beschreibung

Die vorliegende Erfindung betrifft neuartige Derivate von Tris(2-hydroxyphenyl)-methanen, welche als funktionelle Gruppen Polyalkoxygruppen oder mit terminalen hydrophilen Gruppen modifizierte Polyalkoxygruppen aufweisen. Sie betrifft weiterhin die Herstellung derartiger Verbindungen sowie deren Verwendung, insbesondere als Tenside und Verdicker.

Tris(2-hydroxyphenyl)methane sowie verschiedene Derivate davon sind prinzipiell bekannt.

G. Casiraghi, G. Casnati und M. Cornia, Tetrahedron Letters, No. 9, 679 - 682 (1973) beschreiben die Synthese von mono- oder dialkylierten Tris-(2-hydroxyphenyl)-methanen durch Umsetzung entsprechender Phenole mit Triethylorthoformaten.

M. B. Dinger und M. J. Scott beschreiben in Chem. Commun., 1999, 2525/2526, Inorg. Chem. 2000, 39, 1238 - 1254 sowie Inorg. Chem. 2001, 40, 1029 - 1036 die Synthese verschiedener Tris-(3,5-dialkyl-2-hydroxyphenyl)-methane, wobei als Alkylreste Methyl-, t-Butyl- und t-Pentylreste beschrieben werden. Die Trishydroxyverbindungen werden als Komplexbildner für Zink und Alkalimetallionen verwendet.

M. B. Dinger und M. J. Scott, Eur J. Org. Chem. 2000, 2467 - 2478 , beschreiben weiterhin die weitere Umsetzung der OH-Gruppe von Tris-(3,5-dialkyl-2-hydroxyphenyl)-methanen. Die OH-Funktionen können durch Umsetzen mit Halogencarbonsäureestern und Hydrolyse und/oder weiteren Umsetzungen derivatisiert werden. Dinger und Scott beschreiben beispielsweise Tris(3,5-di-t-butyl-2-carboxymethoxyphenyl)methan, Tris(3,5-di-tert-butyl-2-[(dimethylamido)methoxy]phenyl)methan, Tris{3,5-di-tert-butyl-2-[N-(methylglycyl)carbonylmethoxy]phenyl}methan und Tris(3,5-ditert-butyl-2-[(benzylaminocarbonyl)methoxy]phenyl)-methan. Die Derivate können jeweils als Komplexbildner eingesetzt werden, beispielsweise für Zn(II)-Ionen.

K. Matloka, A. Gelis, M. Regalbuto, G. Vandegift und M. J. Scott, Dalton Trans., 2005, 3719 - 3721 bzw. Separation Science and Technology, 41, 2006, 2129 - 2146 sowie M. W. Peters, E. J. Werner und M. J. Scott, Inorg. Chem., 2002, 41, 1701 -1716 offenbaren funktionalisierte Tris-(3,5-dialkyl-2-hydroxyphenyl)-methane, und zwar tripodale Diglykolamide und deren Verwendung zur Komplexierung und Abtrennung von Lanthaniden. Als Zwischenstufe der Synthese werden Tris-(3,5-dialkyl-2-hydroxyphenyl)-methane verwendet, bei denen die OH-Gruppe mit ω-Amino- bzw. Cyanoalkylgruppen verethert ist.

R. Mitra, M.W. Peters und M. Scott, Dalton Trans., 2007, 3924 - 3935, beschreiben weiterhin Tris-(2-hydroxyphenyl)-methan-Derivate, welche terminale 2-Pyridylmethylpiperazin-Gruppen aufweisen. Diese Moleküle können Zinkionen binden und werden als Katalysatoren zur Phosphatdiester- Synthese verwendet. Als Zwischenstufe der mehrstufigen Synthese wird Tris-[2-(2-hydroxylethoxy)-3-methyl-5-t-butylphenyl]-methan offenbart.

EP 597 806 A1 offenbart Cyclohexylgruppen-haltige Glydidylether zur Verwendung als Reaktivverdünner, Flexibilisatoren oder Adhäsionsverbesserer. Als Zwischenprodukt der Synthese werden verschiedene Tris(2-hydroxyphenyl)methane beschrieben, darunter auch solche, bei denen die OH-Funktion mit einer (substituierten) 2-Hydroxyethylgruppe verethert ist.

US 2009/0155714 A1 offenbart Zusammensetzungen zur Herstellung von Photoresists. Als Komponente hierfür werden verschiedene Tris(2-hydroxyphenyl)methan-Derivate verwendet, bei denen die OH-Funktion jeweils mit verschiedenen Carbonsäuren verestert ist.

V. Shvets et al., Journal of Applied Chemistry of USSR, Consultants Bureau, New York, NY, US, Bd. 58, Nr. 6, 1. Januar 1985, 1220 - 1224, offenbart Polyetherderivate zur Herstellung von Emulsionen und Dispersionen.

Es ist bekannt, dass Tenside oberhalb der kritischen Mizellbildungskonzentration (cmc) zu Mizellen aggregieren. Die Form dieser wasserlöslichen Aggregate hängt von der Struktur der Tenside sowie von äußeren Parametern wie Temperatur oder Elektrolytkonzentration ab. Typischerweise können sich oberhalb der Mizellbildungskonzentration sphärische oder stäbchenförmige Mizellen bilden.

Bei bestimmten strukturellen Gegebenheiten und/oder äußeren Parametern können sich auch lange fadenförmige oder wurmartige Mizellen oder Assoziate bilden. Als Folge davon kommt es bereits bei relativ niedriger Tensidkonzentration zu einer Verschlaufung und Überlappung dieser langen Aggregate, wodurch die Viskosität der Tensidlösung deutlich ansteigt. Eine bestimmte zeitliche Mindeststabilität der Mizellen ist dabei Voraussetzung. Dieses temporär gebildete Netzwerk aus Tensidmizellen reagiert aus rheologischer Sicht sowohl viskos als auch elastisch, weshalb man allgemein von viskoelastischen Tensidlösungen spricht. Mizellen setzen einzelne Tenside frei, nehmen Tenside in den Mizellverband auf, zerfallen und bilden sich wieder neu. Tensidmizellen, die viskoelastische Netzwerke ausbilden, sind zeitlich sehr stabil, bevor sie in einzelne Bruchstücke zerfallen und sich wieder neu bilden, so dass das mizellare Netzwerk einer Scherung der Tensidlösung Widerstand entgegensetzen kann und damit sowohl viskos als auch elastisch reagiert. Weitere Einzelheiten zu viskoelastischen, wurmförmige Mizellen bildenden Tensiden wie Hexadecyltrimethylammonium-p-toluolsulfonat oder Cetyl-pyridinium-salicylat sind beispielweise in H. Hoffmann et al., Adv. Colloid Interface Sci. 1982, 17, 275-298 oder M. R. Rojas et al., Journal of Colloid and Interface Science 342 (2010) 103-109) beschrieben.

Aufgrund der dargestellten Eigenschaften eignen sich viskoleastische Tenside ganz besonders als Verdicker und können in verschiedenen Bereichen der Technik eingesetzt werden.

US 2005/0155762 offenbart Betaine mit Alkylketten von 14 bis 24 C-Atomen, beispielsweise Oleylamidopropylbetain oder Erucylamidorpopylbetain als verdickend wirkende, viskoelastische Tenside.

US 7,461,694 B2 offenbart zwitterionische Tenside mit Alkylketten von 16 bis 24 C-Atomen als viskoelastische Tenside.

WO 2008/100436 A1 offenbart eine viskoelastische Tensidmischung aus kationischen, anionischen oder zwitterionischen Tensiden und einem Polymer. Die Tenside weisen Alkylkettenlängen von12 bis 25 C-Atomen auf.

In den zitierten Offenbarungen werden zur Bildung viskoelastischer Tensidlösungen jeweils Tenside mit langen Alkylketten eingesetzt. Ein Nachteil von viskoelastischen Tensiden mit langen Alkylketten ist, dass sie bei Kontakt mit unpolaren Flüssigkeiten diese solubilisieren, wodurch die wurmartigen Mizellen in sphärische Aggregate umgewandelt werden und die Viskoelastizität verloren geht. Außerdem bilden diese viskoelastischen Tenside in Kontakt mit anderen Tensiden in der Regel Mischmizellen, wodurch die Viskoelastizität ebenfalls verloren gehen kann. Strukturen mit kurzen Alkylketten oder Strukturen, die vom üblichen linearen Bauprinzip der Tenside abweichen, bilden in der Regel sphärische Mizelle oder lediglich kurze anisometrische Aggregate und somit keine viskoelastischen Tensidlösungen.

Die bekanntesten viskoelastischen Tenside sind kationische Tenside wie Hexadecyltrimethylammonium-p-toluolsulfonat oder Cetyl-pyridinium-salicylat. Kationische Tenside mit langen Alkylresten sind ökotoxikologisch sehr bedenklich (siehe z.B. Versteeg et al. Chemosphere 24 (1992) 641)). Da sie aufgrund ihrer positiven Ladung an Oberflächen besonders gut adsorbieren, verlieren sie darüber hinaus bei einigen Anwendungen an Wirkung. Daher besteht Bedarf nach Tensiden mit einem günstigeren ökotoxikologischem Profil und geringerer Adsorptionsneigung.

Aufgabe der Erfindung war es, neuartige Verbindungen zu finden, welche insbesondere zur Bildung viskoelastischer Tensidlösungen geeignet sein sollten.

Überraschenderweise wurde gefunden, dass sich durch eine geeignete Derivatisierung von Tris(2-hydroxyphenyl)methan neuartige Verbindungen mit einem neuartigen Eigenschaftsprofil erhalten lassen. Insbesondere lassen sich Tenside erhalten, die zur Herstellung von viskolastischen Tensidlösungen geeignet sind.

Dementsprechend wurden Derivate von Tris(2-hydroxyphenyl)methan gefunden, wobei die Tris(2-hydroxyphenyl)methan-Derivate die allgemeinen Formel (I) 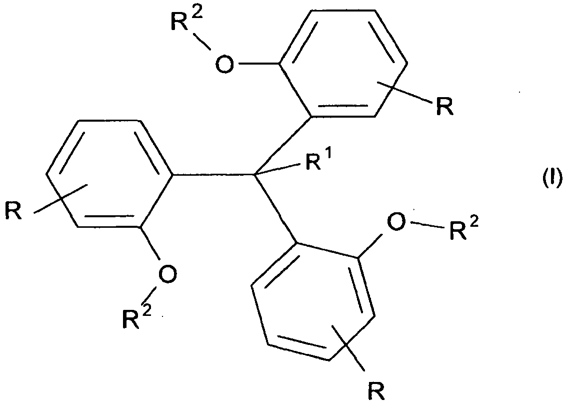 aufweisen, und die Reste R¹, R² und R die nachfolgende Bedeutung haben:
- R:: unabhängig voneinander 0 bis 4 C₁- bis C₃₀-Kohlenwasserstoffreste pro Phenylring,
- R¹:: ein Rest ausgewählt aus der Gruppe von H, OH, F, Cl, Br, I sowie C₁- bis C₃₀-Kohlenwasserstoffgruppen,
- R²:: unabhängig voneinander Reste der allgemeinen Formel -(R⁵-O-)ₙ-R⁶-X (III), wobei n für eine Zahl von 1 bis 50 steht, und wobei die Reste R⁵ unabhängig voneinander ausgewählt werden aus der Gruppe von Resten R⁷, R⁸ und R⁹ 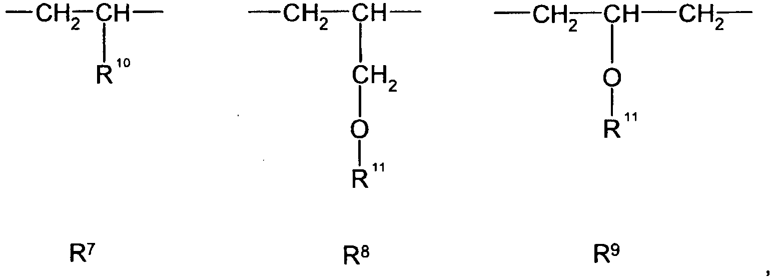 wobei R⁶, X, R¹⁰ und R¹¹ unabhängig voneinander die folgende Bedeutung haben
- R⁶:: eine Einfachbindung oder eine Alkylengruppe mit 1 bis 10 Kohlenstoffatomen, welche optional funktionelle Gruppen als Substituenten aufweisen kann,
- X:: H oder eine hydrophile Gruppe, wobei die hydrophile Gruppe eine saure Gruppe oder ein Rest ist, welcher mindestens eine OH-Gruppe umfasst,
- R¹⁰:: H oder ein C₁- bis C₆-Kohlenwasserstoffrest,
- R¹¹:: eine Gruppe -(R⁵-O-)ₘ-R⁶-X, wobei m für eine Zahl von 0 bis 50 steht,
und wobei die Gesamtzahl z aller Gruppen R⁵ in einem Rest R² 1 bis 50 beträgt, mit der Maßgabe, dass es sich bei z um eine Zahl von 2 bis 50 handelt, falls mindestens ein X für H steht.

Weiterhin wurde die Herstellung derartiger Verbindungen sowie deren Verwendung, insbesondere als Tenside und Verdicker gefunden.

Zu der Erfindung ist im Einzelnen das Folgende auszuführen:

### Erfindungsgemäße Verbindungen

Bei den erfindungsgemäßen Verbindungen handelt es sich um Derivate von Tris(2-hydroxyphenyl)methan der allgemeinen Formel (I).

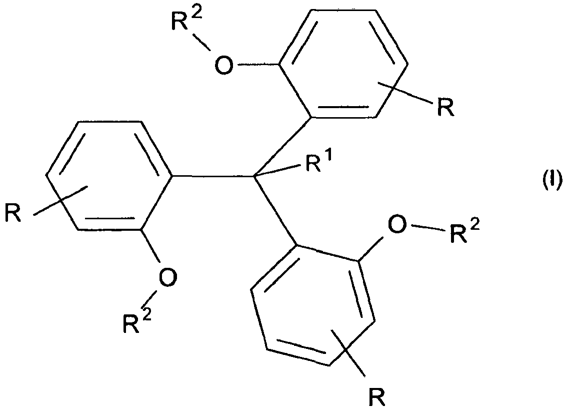

Bei dem Rest R¹ handelt es sich um einen Rest ausgewählt aus der Gruppe von H, OH, F, Cl, Br, I, oder geradkettigen, verzweigten oder cyclischen, aliphatischen und/oder aromatischen C₁- bis C₃₀-Kohlenwasserstoffgruppen. Bevorzugt handelt es sich um H, Cl, eine geradkettige oder verzweigte C₁- bis C₁₂-Alkylgruppe oder eine Benzylgruppe. Besonders bevorzugt handelt es sich bei R¹ um H.

Die drei Phenylringe können jeweils in 3, 4, 5 und 6-Stellung unabhängig voneinander mit Kohlenwasserstoffresten R mit 1 bis 30 Kohlenstoffatomen substituiert sein, wobei die Gruppen beliebig angeordnet sein können. Bevorzugt handelt es sich um 1 oder 2 Gruppen R pro Phenylring. Bei den Gruppen R kann es sich um geradkettige, verzweigte oder cyclische, aliphatische und/oder aromatische Kohlenwasserstoffreste handeln. Bevorzugt handelt es sich bevorzugt um geradkettigte, verzweigte oder cyclische aliphatische Kohlenwasserstoffgruppen mit 1 bis 20, besonders bevorzugt 1 bis 12 Kohlenstoffatomen. Beispiele geeigneter Gruppen R umfassen Methyl-, Ethyl-, n-Propyl-, Isopropyl-, n-Butyl-, i-Propyl-, t-Butyl, n-Pentyl-, 1,1-Dimethylpropyl-, 2,2-Dimethylpropyl, 3-Methylbutyl-, Hexyl-, 2-Ethylhexyl, Heptyl-, Octyl-, Nonyl-, Decyl-, Undecyl-, Dodecyl-, Cyclopentyl-, Cyclohexyl-, Adamantyl- oder Benzylgruppen.

Bevorzugt weisen die erfindungsgemäßen Verbindungen die allgemeine Formel (II) auf.

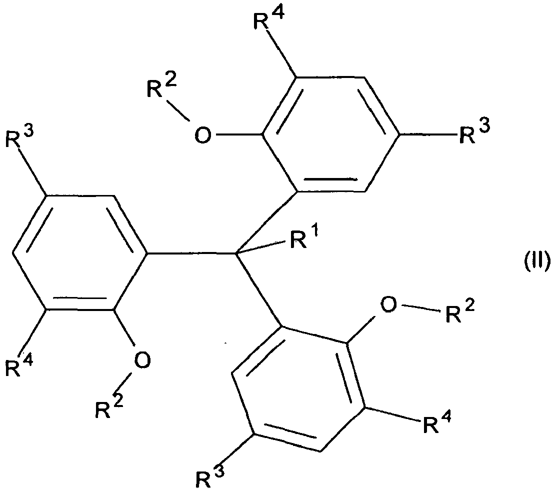

In Formel (II) stehen R³ und R⁴ jeweils unabhängig voneinander für H oder Kohlenwasserstoffreste mit 1 bis 30 C-Atomen, bevorzugt 1 bis 20 C-Atomen, besonders bevorzugt 1 bis 12 C-Atomen. Die Kohlenwasserstoffreste können geradkettig, verzweigt, cyclisch, aliphatisch und/oder aromatisch sein. Bevorzugt handelt es sich um geradkettigte, verzweigte oder cyclische aliphatische Kohlenwasserstoffgruppen mit 1 bis 20, besonders bevorzugt 1 bis 12 Kohlenstoffatomen und ganz besonders bevorzugt um geradkettige oder verzweigte aliphatische Kohlenwasserstoffreste mit 1 bis 6 Kohlenstoffatomen.

Beispiele geeigneter Kohlenwaserstoffgruppen umfassen Methyl-, Ethyl-, Propyl-, Isopropyl-, n-Butyl-, i-Butyl, t-Butyl, n-Pentyl-, 1,1-Dimethylpropyl-, 2,2-Dimethylpropyl, 3-Methylbutyl-, Hexyl-, 2-Ethylhexyl, Heptyl-, Octyl-, Nonyl-, Decyl-, Undecyl-, Dodecyl-, Cyclopentyl-, Cyclohexyl- oder Adamantylgruppen.

Bevorzugt handelt es sich bei R³ und R⁴ um H oder Methyl-, Ethyl-, Propyl-, Isopropyl-, n-Butyl-, i-Butyl, t-Butyl, n-Pentyl-, 1,1-Dimethylpropyl-, 2,2-Dimethylpropyl, 3-Methylbutylgruppen, 1,1,3,3-Tetramethylbutyl, besonders bevorzugt um t-Butylgruppen.

In einer bevorzugten Ausführungsform der Erfindung ist mindestens einer der Reste R³ oder R⁴ nicht H, besonders bevorzugt ist bei dieser Ausführungsform der Rest R³ nicht H. Ganz besonders bevorzugt sind beide Reste R³ und R⁴ jeweils nicht H. Bevorzugte, besonders bevorzugte und ganz besonders bevorzugte Kombinationen von Resten R³ und R⁴ sind in den nachfolgenden Tabellen 1, 2 und 3 genannt:

**Tabelle 1: Liste bevorzugter Kombination**

| R³ | R⁴ |
|---|---|
| t-Butyl | H |
| t-Butyl | Methyl |
| t-Buty | Ethyl |
| t-Butyl | t-Buty |
| Me | Me |
| Me | tBu |
| 1,1-Dimethylpropyl | H |
| 1,1-Dimethylpropyl | Methyl |
| 1,1-Dimethylpropyl | Ethyl |
| 1,1-Dimethylpropyl | t-Butyl |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

**Tabelle 2: Liste besonders bevorzugter Kombinationen**

| R³ | R⁴ |
|---|---|
| t-Butyl | Methyl |
| t-Butyl | t-Butyl |
| 1,1-Dimethylpropyl | Methyl |
| 1,1-Dimethylpropyl | 1,1-Dimethylpropyl |
| 1,1,3,3-Tetramethylbutyl | 1,1,3,3-Tetramethylbutyl |
| t-Buty | 1,1,3,3-Tetramethylbutyl |

**Tabelle 3: Liste ganz besonders bevorzugter Kombinationen**

| R³ | R⁴ |
|---|---|
| t-Buty | t-Buty |
| 1,1-Dimethylpropyl | 1,1-Dimethylpropyl |
| 1,1,3,3-Tetramethylbutyl | 1,1,3,3-Tetramethylbutyl |
| t-Butyl | 1,1,3,3-Tetramethylbutyl |

Ganz besonders bevorzugt handelt es sich sowohl bei R³ als auch bei R⁴ um t-Butylreste.

Bei den Resten R² in den oben genannten Formeln (I) und (II) handelt es sich unabhängig voneinander um Reste der allgemeinen Formel -(R⁵-O-)ₙ-R⁶-X (III).

Bei den Resten R⁵ in Formel (III) handelt es sich unabhängig voneinander um Gruppen ausgewählt aus der Gruppe von Resten R⁷, R⁸ und R⁹

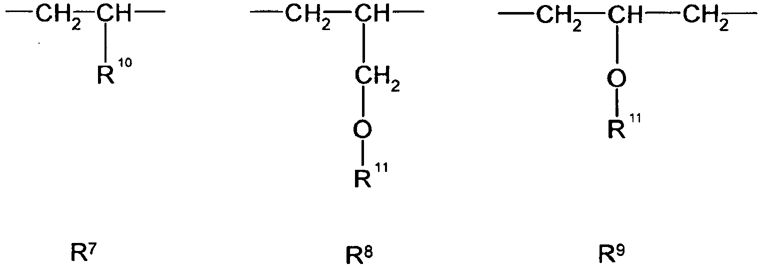

Hierbei stehen in den Gruppen R⁷ die Reste R¹⁰ unabhängig voneinander für H oder einen Kohlenwasserstoffrest mit 1 bis 6 Kohlenstoffatomen, bevorzugt einen aliphatischen Kohlenwasserstoffrest mit 1 bis 6 Kohlenstoffatomen. Anstelle der in Formel (III) dargestellten Orientierung -CH₂-CH(R¹⁰)- kann die Alkylengruppe auch in inverser Orientierung -CH(R¹⁰)-CH₂- in die Polyoxyalkylenkette eingebaut sein. Die Formel (III) soll beide Orientierungen umfassen, wobei selbstverständlich auch beide Orientierungen in einer Ketten enthalten sein können. Beispiele für Reste R¹⁰ umfassen H sowie Methyl-, Ethyl-, n-Propyl oder Phenylreste. Insbesondere handelt es sich bei R¹⁰ um H, Methyl-, Ethyl- oder n-Propylreste, bevorzugt um H, Methyl- oder Ethylreste, besonders bevorzugt um H oder Methyl.

In den Gruppen R⁸ und R⁹ stehen die Gruppen R¹¹ für Gruppen der allgemeinen Formel -(R⁵-O-)ₘ-R⁶-X, wobei m für eine Zahl von 0 bis 50 steht. Es kann sich also (für den Fall m = 0) um eine Gruppe -R⁶-X handeln, welche direkt mit dem O-Atom verknüpft ist, oder (für den Fall m > 0) um eine Gruppe, bei der -R⁶-X über eine Polyalkoxygruppe mit dem O-Atom verknüpft ist. Aufgrund der Tatsache, dass in letzterem Falle R¹¹ selbst wieder Gruppen R⁸ und R⁹ enthalten kann, können die Gruppen R² auch mehrfach verzweigt sein.

Die Reste R⁷, R⁸ und R⁹ können beliebig in der Resten R² angeordnet werden, beispielsweise statistisch, blockweise, alternierend oder mit einem Gradienten.

Je nach Art des Restes R², können nur eine Gruppe X oder auch mehrere Gruppen X in einem Rest R² enthalten sein. Bei hydrophilen Gruppen kann es sich insbesondere um saure Gruppen handeln, bevorzugt um eine Gruppe ausgewählt aus der Gruppe von Carboxylgruppen -COOM, Sulfonsäuregruppen -SO₃M, Sulfatgruppen -OSO₃M, Phosphonsäuregruppen -PO₂M₂ oder Phoshorsäuregruppen -OPO₃M₂, für wobei M für H⁺ oder ein k-wertiges Gegenion ¹/ₖY^{k+} steht. Die sauren Gruppen können also als freie Säure vorliegen und/oder als ein Salz davon. Sofern es sich bei M nicht um H⁺ handelt, handelt es sich bevorzugt um ein einwertiges Gegenion, wie beispielsweise NH₄⁺-, Ammoniumionen mit organischen Resten oder Alkalimetallionen. Bevorzugte saure Gruppen sind solche ausgewählt aus der Gruppe von Carboxylgruppen -COOM, Sulfonsäuregruppen -SO₃M oder Sulfatgruppen -OSO₃M, besonders bevorzugt Sulfatgruppen -OSO₃M.

Bevorzugte hydrophile Gruppen umfassen weiterhin Reste, welche mindestens eine, bevorzugt mindestens 2 OH-Gruppen umfassen, insbesondere Mono- oder Oligosaccharidreste, bevorzugt Monosaccharidreste. Es kann sich prinzipiell um alle Arten von Sacchariden handeln. Bevorzugt können von Pentosen und Hexosen, insbesondere von Hexosen abgeleitete Reste eingesetzt werden. Beispiele geeigneter Monosaccharide umfassen Glucose, Mannose, Galaktose, Fruktose oder Ribose. Bevorzugt können von Glukose abgeleitete Rest eingesetzt werden. Es kann sich auch um Derivate der Saccharide handeln, beispielsweise um durch Reduktion oder Oxidation aus den Sacchariden hervorgehende Produkte. Insbesondere kann es sich bei derartigen Derivaten um Zuckersäuren wie beispielsweise Gluconsäure handeln.

Beispiele anderer hydrophiler Gruppen umfassen beispielsweise Aminoxidgruppen.

Bei R⁶ handelt es sich um eine Einfachbindung oder eine Alkylengrupe mit 1 bis 10 Kohlenstoffatomen, bevorzugt 1 bis 3 Kohlenstoffatomen, welche optional funktionelle Gruppen als Substituenten, insbesondere eine OH-Gruppe aufweisen kann. Beispiele derartiger Gruppen umfassen -CH₂-, -CH₂CH₂-, -CH₂-CH₂-CH₂- oder -CH₂-CH(OH)-CH₂-Gruppen.

Die Gesamtzahl aller Gruppen R⁵ in einem Rest R², d.h. der Gruppen R⁵ in der Hauptgruppe als auch der Gruppen R⁵ in eventuell vorhandenen Verzweigungen, soll nachfolgend mit z bezeichnet werden. Für den Fall, dass es sich um eine lineare Gruppe R² handelt, entspricht z der Zahl n.

Erfindungsgemäß handelt es sich bei z um eine Zahl von 2 bis 50 für den Fall, dass mindestens ein X für H steht, und es handelt sich um eine Zahl von 1 bis 50 für den Fall, dass kein X für H steht. Die Zahlen z, n und m beziehen sich dabei in bekannter Art und Weise auf den Mittelwert der im Molekül vorhandenen Alkoxygruppen, wobei der Mittelwert selbstverständlich nicht eine natürliche Zahl sein muss, sondern auch eine positive rationale Zahl sein kann. In der Regel steht z-unabhängig von der Natur des Substituenten X für eine Zahl von 2 bis 50, insbesondere 4 bis 40, bevorzugt 5 bis 30, besonders bevorzugt 8 bis 20 und ganz besonders bevorzugt 10 bis 15.

Der Fachmann trifft unter den möglichen Gruppen (III) sowie der Reste R¹, R², R³ und R⁴ je nach dem gewünschten Verwendungszeck der Verbindungen eine entsprechende Auswahl.

In einer ersten bevorzugten Ausführungsform der Erfindung handelt es sich bei mindestens einem der Reste R⁵ um einen Rest R⁷. In einer besonders bevorzugten Ausführungsform der Erfindung handelt es sich bei allen Resten R⁵ um Reste R⁷. Sofern die Reste R⁷ verschiedene Reste R¹⁰ umfassen, können diese auf beliebige Art und Weise angeordnet werden, beispielsweise statistisch, blockweise, alternierend oder mit einem Gradienten. Bevorzugt handelt es sich zumindest bei einem Teil der z Reste R¹⁰ um H, insbesondere bei mindestens 20 %, bevorzugt bei mindestens 50 %, besonders bevorzugt bei mindestens 80 % und ganz besonders bevorzugt handelt es sich bei allen Resten R¹⁰ um H.

In einer zweiten bevorzugten Ausführungsform der Erfindung handelt es sich bei den Resten R² um Gruppen -(R⁷-O-)ₙ-R⁶-X (IIIa). Reste R⁸ und Reste R⁹ sind bei dieser Ausführungsform nicht vorhanden. Besonders bevorzugt handelt es sich um Gruppen - (R⁷-O-)ₙ-H (IIIb), d.h. X = H und R⁶ ist eine Einfachbindung. In (IIIa) und (IIIb) steht n für eine Zahl 5 bis 30, besonders bevorzugt 8 bis 20 und ganz besonders bevorzugt 10 bis 15. Bei den Resten R¹⁰ in den Resten R⁷, d.h. den Resten -CH₂-CH(R¹⁰)-, handelt es sich unabhängig voneinander um einen Rest ausgewählt aus der Gruppe von H, Methyl oder Ethyl, wobei es sich bei mindestens 50 % der Reste (R¹⁰) um H handelt, bevorzugt bei mindestens 80 % der Reste und ganz besonders bevorzugt handelt es sich bei allen Resten R¹⁰ um H. Sofern mehrere verschiedene Reste R¹⁰ vorhanden sind, können diese statistisch, alternierend oder blockweise angeordnet sein. Im Falle von blockweiser Anordnung ist es bevorzugt, wenn die Ethylenoxidgruppen (also R¹⁰ = H) den terminalen Block bilden.

In einer dritten bevorzugten Ausführungsform der Erfindung handelt es sich bei den Resten R² um Gruppen -(-CH₂-CH(R¹²)-O-)ₐ-(-CH₂-CH₂-O-)_{b}-H (IIIc), wobei die beiden Blöcke in dieser Reihenfolge angeordnet sind. Es handelt sich also wiederum um eine Ausführungsform, in der nur Gruppen R⁷ vorhanden sind, X ist H und R⁶ ist eine Einfachbindung. Bei den Resten R¹² in der Formel (IIIc) handelt es sich um Kohlenwasserstoffreste mit 1 bis 6 Kohlenstoffatomen, bevorzugt aliphatische Kohlenwasserstoffreste mit 1 bis 6 Kohlenstoffatomen, bevorzugt eine Methyl und/oder eine Ethylgruppe. Bei a handelt es sich um Zahlen von 1 bis 49, bevorzugt 1 bis 10 und bei b um Zahlen von 1 bis 49, bevorzugt 1 bis 20 mit der Maßgabe, dass a + b = n = 2 bis 50. Bevorzugt ist b ≥ a.

In einer vierten bevorzugten Ausführungsform der Erfindung handelt es sich bei den Resten R² um Gruppen -(-CH₂-CH(R¹³)-O-)ₙ-R⁶-X (IIId), wobei bei mindestens einer der Gruppen R² der Rest X nicht für H steht. Bei den Resten R¹⁰ in der Formel (IIId) handelt es sich unabhängig voneinander um einen Rest ausgewählt aus der Gruppe von H, Methyl oder Ethyl, wobei es sich bei mindestens 20 % der Reste R¹⁰ um H handelt, bevorzugt bei mindestens 50 % der Reste, besonders bevorzugt bei mindestens 80 % der Reste und besonders bevorzugt handelt es sich bei allen Resten R¹⁰ um H. Sofern mehrere verschiedene Reste R¹³ vorhanden sind, können diese statistisch, alternierend oder blockweise angeordnet sein. Im Falle von blockweiser Anordnung ist es bevorzugt, wenn die Ethylenoxidgruppen (also R¹³ = H) den terminalen Block bilden. Bevorzugt handelt es sich bei X in Formel (IIIc) um Carboxylgruppen -COOM, Sulfonsäuregruppen -SO₃M, Sulfatgruppen -OSO₃M. Beispielsweise kann es sich um Gruppen -(-CH₂-CH(R¹³)-O-)ₙ-H in Kombination mit Gruppen -(-CH₂-CH(R¹³)-O-)ₙ-SO₃H handeln. Bei n handelt es sich in dieser Ausführungsform bevorzugt um Zahlen von n = 1 bis 30.

In einer fünften bevorzugten Ausführungsform der Erfindung umfassen die Reste R² der allgemeinen Formel -(R⁵-O-)ₙ-R⁶-X (III) Reste R⁸ und/oder R⁹. Es kann sich sowohl um Reste handeln, welche ausschließlich Gruppen R⁸ und/oder R⁹ umfassen, als auch um solche, welche neben R⁸ und/oder R⁹ zusätzlich Gruppen R⁷ umfassen. Bevorzugt handelt es sich bei R⁷ um Gruppen R⁷ mit R¹⁰ = H, d.h. vom Ethylenoxid abgeleiteten Gruppen.

In einer sechsten bevorzugten Ausführungsform der Erfindung handelt es sich bei R² um Reste der allgemeinen Formel -(-CH₂CH(CH₂OH)-O-)_{c}(CH₂CH(R¹⁴)-O-)_{d}-R⁶-X (IIIe), wobei die Summe aus c+d = z. In der Formel (IIIe) handelt es sich um Monomere R⁷ und R⁸, wobei R¹¹ für H steht. Bei den Resten R¹⁴ in der Formel (IIIe) handelt es sich unabhängig voneinander um einen Rest ausgewählt aus der Gruppe von H, Methyl oder Ethyl, wobei es sich bei mindestens 20 % der Reste R¹⁰ um H handelt, bevorzugt bei mindestens 50 % der Reste, besonders bevorzugt bei mindestens 80 % der Reste und besonders bevorzugt handelt es sich bei allen Resten R¹⁴ um H. Bei R⁶ handelt es sich bei dieser Ausführungsform bevorzugt um eine Einfachbindung und bei X bevorzugt um H.

In einer weiteren Ausführungsform der Erfindung handelt es sich um Derivate von Tris(2-hydroxyphenyl)methanen, welche erhältlich sind, indem man Carbonsäurevinylester in Gegenwart von Verbindungen der allgemeinen Formeln (I) oder (II) radikalisch polymerisiert. Die Polymerisation von Carbonsäurevinylestern in Gegenwart Polyalkoxygruppen ist dem Fachmann prinzipiell bekannt. Bei dieser Reaktion pfropfen Vinylester-, Oligovinylester- und/oder Polyvinylestergruppen auf die Polyalkoxygruppen auf, d.h. es entstehen zusätzliche Seitengruppen aufweisende Polyalkoxygruppen. Die Polyvinylestergruppen werden anschließend zumindest teilweise zu OH-Gruppen hydrolysiert. Die OH-Gruppen können anschließend optional mit Gruppen -R⁶-X funktionalisiert werden. Das Reaktionsschema ist nachfolgend beispielhaft illustriert.

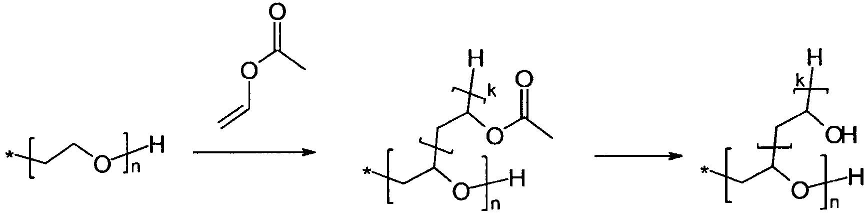

Geeignete Carbonsäurevinylester umfassen insbesondere Vinylformiat, Vinylacetat und Vinylpropionat. Zur Modifizierung besonders geeignet sind insbesondere Verbindungen, welche Gruppen (IIIb) oder (IIIc) aufweisen.

### Herstellung der erfindungsgemäßen Verbindungen

Zur Herstellung der erfindungsgemäßen Verbindungen können zunächst einmal Tris(2-hydroxyphenyl)methan-Verbindungen der allgemeinen Formeln (IV) oder (V) mit dem gewünschten Substitutionsmuster hinsichtlich R¹, und R bzw. R³ und R⁴ synthetisiert werden.

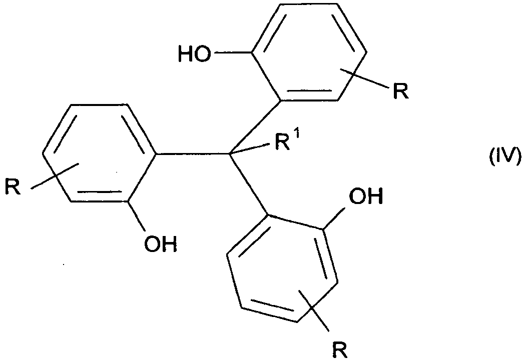

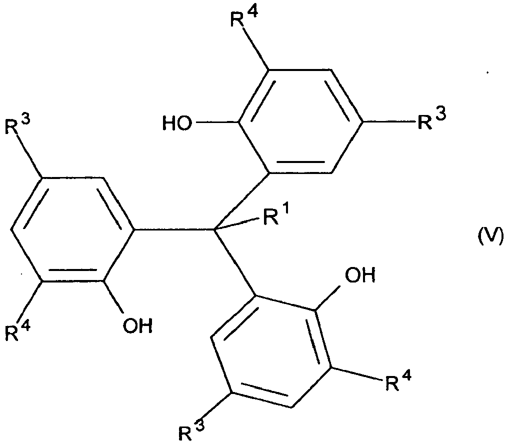

Die Methoden zu Herstellung der Verbindungen sind in der eingangs zitierten Literatur, beispielsweise G. Casiraghi, G. Casnati und M. Cornia, Tetrahedron Letters, No. 9, 679 - 682 (1973), M. B. Dinger und M. J. Scott, Chem. Commun., 1999, 2525/2526, Inorg. Chem. 2000, 39, 1238 - 1254 sowie Inorg. Chem. 2001, 40, 1029 - 1036, M. B. Dinger und M. J. Scott, Eur J. Org. Chem. 2000, 2467 - 2478 , K. Matloka, A. Gelis, M. Regalbuto, G. Vandegift und M. J. Scott, Dalton Trans., 2005, 3719 - 3721, M. W. Peters, E. J. Werner und M. J. Scott, Inorg. Chem., 2002, 41, 1701 - 1716 und R. Mitra, M.W. Peters und M. Scott, Dalton Trans., 2007, 3924 - 3935 ausführlich beschreiben.

Die Tris(2-hydroxyphenyl)methan-Verbindungen der allgemeinen Formeln (IV) oder (V) können in einem zweiten Schritt in prinzipiell bekannter Art und Weise alkoxyliert werden.

Die Durchführung von Alkoxylierungen ist dem Fachmann prinzipiell bekannt. Es ist dem Fachmann ebenfalls bekannt, dass man durch die Reaktionsbedingungen, insbesondere die Wahl des Katalysators, die Molekulargewichtsverteilung der Alkoxylate beeinflussen kann.

Zur Alkoxylierung werden C₂- bis C₈-Alkylenoxide eingesetzt, beispielsweise, Ethylenoxid, Propylenoxid, Butylenoxid oder Styroloxid. Bevorzugt sind jeweils die 1,2-Alkylenoxide. Die Verwendung dieser Alkylenoxide führt zu Gruppen, welche Reste R⁷ umfassen.

Um Reste R⁸ und R⁹ zu erhalten, setzt man Glycidol (VIa) oder mit einer geeigneten Schutzgruppe R¹⁵ geschütztes Glycidol (Vlb) 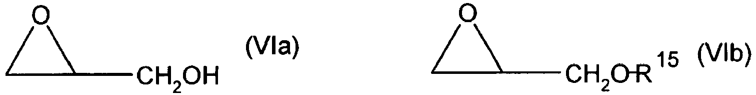 als Alkenoxid ein. Bei R¹⁵ kann es sich prinzipiell um alle Arten von Gruppen handeln, mit denen sich die OH-Funktion während der Alkoxylierung schützen lässt. Sie können nach der Alkoxylierung oder auch erst nach Einführung der Gruppen -R⁶-X in prinzipiell bekannter Art und Weise abgespalten werden. Bei R¹⁵ kann es sich beispielsweise um eine t-Butylgruppe oder eine Benzylgruppe handeln.

Da nach der Reaktion des ungeschützten Glycidols zwei OH-Gruppen in der Kette vorhanden sind, welche weiterreagieren können, entstehen die bereits zitierten Verzweigungen im Rest R². Mithilfe des geschützten Glycidols lassen sich gezielt Gruppen R⁸ der Formel -CH₂-CH(CH₂OH)- in den Rest R² einführen.

Bei der Alkoxylierung kann es sich um eine basenkatalysierte Alkoxylierung handeln. Dazu können die Tris(2-hydroxyphenyl)methan-Verbindungen in einem Druckreaktor mit Alkalimetallhydroxiden, bevorzugt Kaliumhydroxid oder mit Alkalialkoholaten wie beispielsweise Natriummethylat versetzt werden. Durch verminderten Druck (bspw. <100 mbar) und/oder Erhöhung der Temperatur (30 bis 150°C) kann noch in der Mischung vorhandenes Wasser abgezogen werden. Der Alkohol liegt danach als das entsprechende Alkoholat vor. Anschließend wird mit Inertgas (z.B. Stickstoff) inertisiert und das oder die Alkylenoxid(e) bei Temperaturen von 60 bis 180°C bis zu einem Druck von max. 10 bar schrittweise zugegeben. Am Ende der Reaktion kann der Katalysator durch Zugabe von Säure (z.B. Essigsäure oder Phosphorsäure) neutralisiert und kann bei Bedarf abfiltriert werden. Optional kann die Alkoxylierung auch in Gegenwart eines Lösungsmittels durchgeführt werden. Dies kann z.B. Toluol, Xylol, Dimethylformamid oder Ethylencarbonat sein.

Die Alkoxylierung der Alkohole kann aber auch mittels anderer Methoden vorgenommen werden, beispielsweise durch säurekatalysierte Alkoxylierung. Weiterhin können beispielsweise Doppelhydroxidtone wie in DE 43 25 237 A1 beschrieben eingesetzt werden, oder es können Doppelmetallcyanid-Katalysatoren (DMC-Katalysatoren) verwendet werden. Geeignete DMC-Katalysatoren sind beispielsweise in der DE 102 43 361 A1, insbesondere den Abschnitten [0029] bis [0041] sowie der darin zitierten Literatur offenbart. Beispielsweise können Katalysatoren vom Zn-Co-Typ eingesetzt werden. Zur Durchführung der Reaktion kann der Alkohol R-OH mit dem Katalysator versetzt, die Mischung wie oben beschrieben entwässert und mit den Alkylenoxiden wie beschrieben umgesetzt werden. Es werden üblicherweise nicht mehr als 1000 ppm Katalysator bezüglich der Mischung eingesetzt, und der Katalysator kann aufgrund dieser geringen Menge im Produkt verbleiben. Die Katalysatormenge kann in der Regel geringer sein als 1000 ppm, beispielsweise 250 ppm und weniger.

Die Alkoxylierung kann alternativ auch durch Reaktion der Verbindungen (IV) und (V) mit cyclischen Carbonaten wie beispielsweise Ethylencarbonat vorgenommen werden.

Mittels der Alkoxylierung erhält man bereits erfindungsgemäße Verbindungen, nämlich solche mit X = H. Diese weisen terminale OH-Gruppen auf. Dies ist in den nachfolgenden Abbildungen (VII) bzw. (VIII) beispielhaft anhand von erfindungsgemäßen Verbindungen, welche Polyalkoxyketten aus Gruppen R⁷ aufweisen gezeigt.

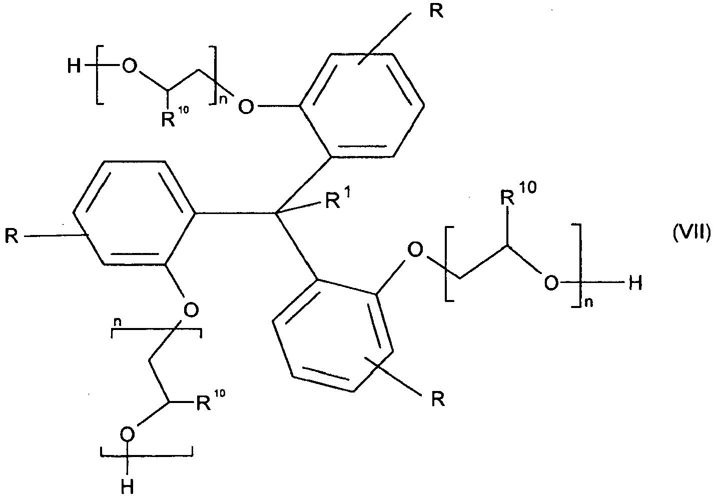

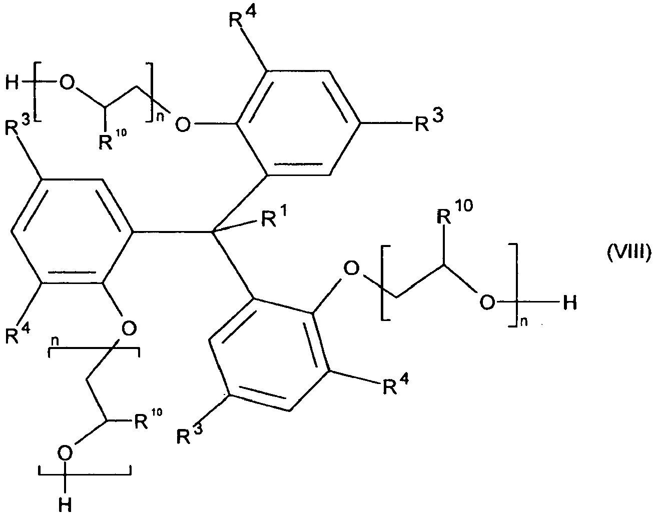

Zur Einführung von Gruppen X, welche nicht H sind, werden die alkoxylierten, terminale OH-Gruppen aufweisenden Tris(2-hydroxyphenyl)methan-Derivate der Formel (VII) bzw. (VIII) auf geeignete Art und Weise weiter mit Gruppen -R⁶-X funktionalisiert. Hierdurch werden Verbindungen der allgemeinen Formel (IX) bzw. (X) erhalten. 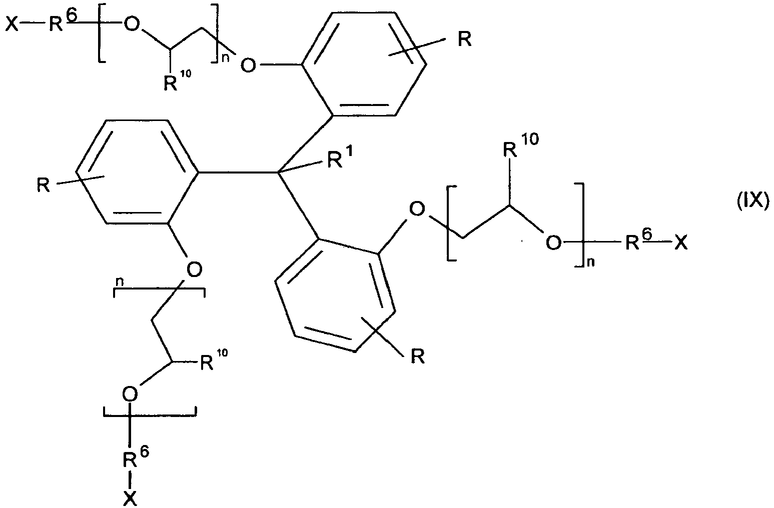 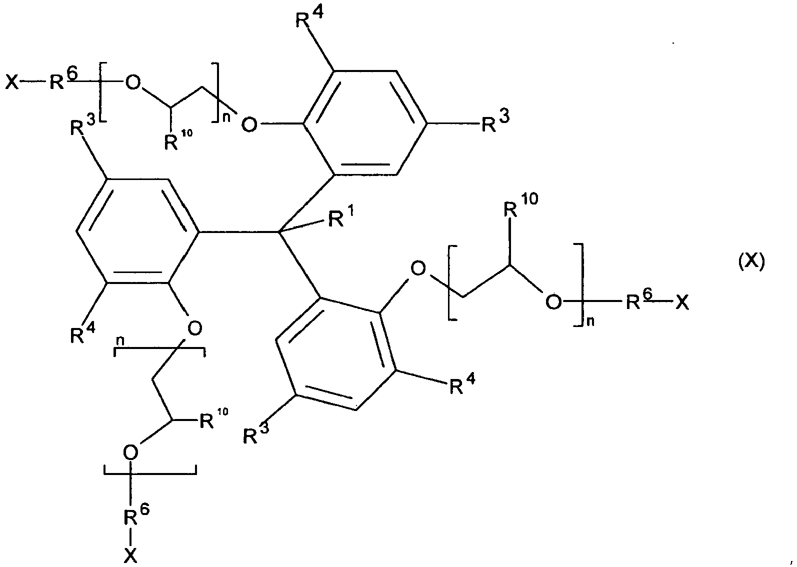 Sulfatgruppen -OSO₃M umfassende Derivate können erhalten werden durch Umsetzung der terminalen OH-Gruppen mit SO₃, Schwefelsäure, Chlorschwefelsäure oder Aminosulfonsäure (CAS-Nr. 5329-14-6) und nachfolgender Neutralisation mit z.B. Natronlauge. Dies kann z.B. in einem Fallfilmreaktor durchgeführt werden. Durch diese Reaktion werden nur die terminalen OH-Gruppen durch Sulfatgruppen substituiert. R⁶ steht bei dieser Reaktion für eine Doppelbindung.

Sulfonatgruppen -SO₃M umfassende Derivate können erhalten werden durch Substitution der OH-Gruppe gegen Cl unter Verwendung von Phosgen oder Thionylchlorid. Die Umsetzung kann in Gegenwart eines Lösungsmittels wie z.B. Chlorbenzol vorgenommen werden. Dabei frei werdende HCl sowie freiwerdendes CO₂ oder SO₂ kann vorteilhaft durch Strippen mit Stickstoff aus dem System entfernt werden, so dass eine Etherspaltung unterbunden wird. Die Alkylalkoxychlor-Verbindung wird im Anschluss daran mit einer wässrigen Lösung von Natriumsulfit umgesetzt, wobei das Chlorid durch Sulfit substituiert wird und das Sulfonat erhalten wird. Die Substitution kann in Gegenwart eines Phasenvermittlers (bspw. C₁- bis C₈-Alkohole) bei einer Temperatur 100 - 180°C und Druck vorgenommen werden. Die Sulfonate können alternativ durch Addition von Vinylsulfonsäure an die Verbindung (V) erhalten werden. Einzelheiten hierzu sind beispielsweise in EP 311 961 A1 beschrieben. Sulfonate können weiterhin erhalten werden, indem man die Verbindungen (V) mit 1,3-Propansulton oder 1,4-Butansulton umsetzt. Hierbei werden Sulfonate mit einer terminalen Gruppe -CH₂-CH₂-CH₂-SO₃M (d.h. R⁶ = CH₂-CH₂-CH₂-) bzw. -CH₂-CH₂-CH₂-CH₂-SO₃M (d.h. R⁶ = CH₂-CH₂-CH₂-CH₂-) erhalten. Verbindungen mit einer terminalen Gruppe -CH₂-CH(OH)-CH₂-SO₃M (d.h. R⁶ = -CH₂-CH(OH)-CH₂-) lassen sich durch Umsetzung der Verbindung (V) mit Epichlorhydrin und nachfolgender nucleophiler Substitution der Chloridgruppe durch Natriumsulfit erhalten.

Carboxylatgruppen -COOM umfassende Derivate können durch Oxidation der Verbindung (V) erhalten werden. Hierzu sind alle Oxidationsmittel geeignet, gegebenenfalls in Verbindung mit geeigneten Katalysatoren, welche die terminale OH-Gruppe der Verbindung (V) zur COOH-Gruppe oxidieren können, ohne in großem Maße andere Teile des Moleküls zu oxidieren. Die Oxidation kann beispielsweise mit Hilfe von Luft oder Sauerstoff unter Verwendung eines Edelmetallkatalysators (beispielsweise eines Katalysators auf Basis von Palladium) vorgenommen werden. Bei dieser Synthesevariante wird eine terminale Gruppe -CH₂-COOM erhalten (d.h. R⁶ = -CH₂-). Carboxylate können weiterhin auch hergestellt werden, indem man an die OH-Gruppen mittels einer Michael-Addition (Meth)acrylsäure oder einen (Meth)acrylsäureester addiert. Falls die Ester verwendet werden, werden diese nach der Addition verseift. Bei dieser Synthesevariante werden -je nachdem, ob Acrylsäure oder (Meth)acrylsäure bzw. deren Ester verwendet wurden- terminale Gruppen -CH₂-CH₂-COOM oder -CH₂-CH(CH₃)-COOM erhalten.

Phosphatgruppen lassen sich durch Umsetzung mit Phosphorpentoxid einführen, Phosphonatgruppen durch Umsetzung mit Vinylphosphonsäure.

Verbindungen mit Mono- oder Oligosaccharidgruppen können hergestellt werden, indem man das entsprechend Saccharid, beispielsweise Glucose mit Hilfe eines sauren Katalysators, wie beispielsweise para-Toluolsulfonsäure und n-Butanol in das entsprechende Butylacetal überführt. Das entstehende Reaktionswasser kann durch Anlegen von Vakuum aus dem Reaktionsgemisch entfernt werden. Im Anschluss wird die Verbindung (V) zugegeben und die Umacetalisierung durch destillative Entfernung des Butanols aus dem Gleichgewicht vorangetrieben. Der saure Katalysator kann am Ende der Reaktion durch Zugabe von Base, beispielsweise NaOH oder KOH neutralisiert werden.

Je nach Art der Gruppen R² weisen die erhaltenden Verbindungen nur eine terminale Gruppe -R⁶-X auf oder auch mehrere terminale und/oder seitenständige Gruppen -R⁶-X.

Bei der Einführung der terminalen Gruppe -R⁶-X müssen selbstverständlich nicht alle OH-Gruppen der H-terminierten erfindungsgemäßen Verbindungen umgesetzt werden. Es ist möglich, nur einen Teil der Gruppen, beispielsweise nur durchschnittlich jede dritte Gruppe umzusetzen. Auf diese Art und Weise lassen sich die Eigenschaften der erfindungsgemäßen Verbindungen an den gewünschten Verwendungszweck anpassen.

Bei Verwendung von Glycidol sind verschiedene Synthesevarianten denkbar. Verwendet man ungeschütztes Glycidol, so können die Gruppen R² verzweigt sein und mehrere terminale oder seitenständige OH-Gruppen aufweisen. Diese Gruppen können ganz oder auch nur teilweise zur Gruppen -R⁶-X umgesetzt werden. Bei einer nur teilweisen Umsetzung erfolgt die Umsetzung statistisch.

Verwendet man geschütztes Glycidol (VIb) so entsteht zunächst einmal eine unverzweigte Polyalkoxykette mit einer terminalen OH-Gruppe und seitenständigen, geschützten OH-Gruppen. Man kann nun einerseits die Schutzgruppen zunächst abspalten und dann die Einführung der Gruppen -R⁶-X vornehmen. In diesem Falle entsteht eine lineare Gruppe R², welche end- und/oder seitenständig Gruppen -R⁶-X aufweist. Spaltet man in einer alternativen Synthese die Schutzgruppen zunächst nicht ab, sondern nimmt erst die Einführung der Gruppen -R⁶-X vor, dann reagieren nur die terminalen OH-Gruppen. Die Abspaltung der Schutzgruppen kann danach erfolgen. In diesem Falle entsteht eine Gruppe R², welche eine terminale Gruppe -R⁶-X aufweist und darüber hinaus seitenständige Methylolgruppen -CH₂OH.

### Verwendung der erfindungsgemäßen Verbindungen

Die neuen Verbindungen eignen sich zur Verwendung als Tenside. Sie eignen sich insbesondere zur Herstellung von viskoelastischen Tensidlösungen und können daher als Komponente von verdickenden Formulierungen verwendet werden. Als Tenside eignen sich insbesondere Verbindungen der allgemeinen Formel (II), bei denen die Reste R³ und R⁴ nicht für H stehen, und bei denen die Reste R² die allgemeinen Formeln (IIIb), (IIIc) oder (IIId) aufweisen. Sie erniedrigen die Oberflächenspannung von Wasser auf für Tenside typische Werte von unter 35 mN/m und bilden Mizellen bereits bei Konzentrationen unter 0,1 g/l. Die gebildeten Mizellen sind sehr stabil, die Tenside demzufolge wenig dynamisch.

Die neuen Verbindungen eignen sich aufgrund ihrer grenzflächenaktiven Eigenschaften aber auch aufgrund ihrer verdickenden Wirkung weiterhin beispielsweise zum Einsatz in Wasch- und Reinigungsmitteln, Farben und Anstrichmittel, in kosmetischen und pharmazeutischen Formulierungen, Papier-, Textil- und Lederhilfsmitteln, Formulierungen für die Human- und Tierernährung, den Bausektor, Pflanzenschutzformulierungen sowie allgemein zur Herstellung von Emulsionen und Dispersionen. Gegenüber unpolaren Flüssigkeiten erniedrigen diese Tenside die Öl/Wasser-Grenzflächenspannung abhängig von der Art des verwendeten Öls und den Resten R³ bis R⁴ auf Werte von unter 5 mN/m.

Besonders gut als Tenside geeignet ist beispielsweise Tris-(3,5-di-tert-butyl-2-hydroxyphenyl)-methan umgesetzt mit Ethylenoxid, insbesondere mit 9 bis 14 EO-Einheiten pro Polyalkoxygruppe.

Die nachfolgenden Beispiele sollen die Erfindung näher illustrieren:

### I) Synthese der erfindungsgemäßen Verbindungen

### Synthesebeispiel 1:

### Synthese von Tris-(3,5-di-tert-butyl-2-hydroxyphenyl)-methan

Tris-(3,5-di-tert-butyl-2-hydroxyphenyl)-methan (CAS-No.143560-44-5) wurde mittels des von M. B. Dinger, M. J. Scott, Eur. J. Org. Chem. 2000, 2467 beschriebenen Verfahrens hergestellt. Tris-(3,5-di-*tert*-butyl-2-hydroxyphenyl)-methan wird nachfolgend auch als TRIS abgekürzt.

### Synthesebeispiel 2:

### Synthese von TRIS[(-CH₂-CH₂-O)₁₁H]₃ durch Ethoxylierung von TRIS mit 33 EO-Einheiten

In einem 2l Autoklaven wird TRIS (100g) in Toluol (150 ml) suspendiert mit Kalium-*tert-*butylat (3.0 g) versetzt. Es wird dreimal mit N₂ gespült, ein Vordruck von ca. 1.3 bar N₂ eingestellt und die Temperatur auf 119-121°C erhöht. Ethylenoxid (231 g) wird so zudosiert, dass die Temperatur zwischen 119°C-130°C bleibt. Anschließend wird 16 h bei 90°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Man erhält 263 g Zwischenprodukt, das eine OH-Zahl von 105,9 mg/g aufweist. In einem zweiten Schritt werden 239 g des so erhaltenen Zwischenproduktes in einem 2l Autoklaven mit Kalium-*tert*-butylat (1,5 g) versetzt. Es wird dreimal mit N₂ gespült, ein Vordruck von ca. 1.3 bar N₂ eingestellt und die Temperatur auf 119-121 °C erhöht. Ethylenoxid (231 g) wird so zudosiert, dass die Temperatur zwischen 119°C-130°C bleibt. Anschließend wird 16 h bei 100°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Auf diese Weise erhält man 307 g der Zielverbindung. OH-Zahl: 87,5 mg/g. Das TAI-¹H-NMR (Lit.: J. Loccufier, M. van Bos, E. Schacht, Polymer Bulletin, 1991, 27, 201) der Substanz zeigt einen mittleren Ethoxylierungsgrad von 11 Ethylenoxid-Einheiten pro phenolischer OH-Gruppe.

### Synthesebeispiel 3:

### Synthese von TRIS[(-CH₂-CH₂-O)₁₁H]₂[(-CH₂-CH₂-O)₁₁-SO₃Na] durch Sulfatierung

Das in Beispiel 2 synthetisierte Produkt TRIS[(-CH₂-CH₂-O)₁₁H]₃ wird in Dichlormethan (45 g) gelöst und auf 5-10°C abgekühlt. Danach wird Chlorsulfonsäure (2,12 g) so zugetropft, dass die Temperatur 10°C nicht übersteigt. Man lässt 1 h bei 10°C, danach über Nacht bei Raumtemperatur rühren, bevor obiges Reaktionsgemisch in eine wässrige NaOH-Lösung (50%, 1,98 g) bei max. 10°C getropft wird. Das Dichlormethan wird unter leichtem Vakuum am Rotationsverdampfer bei 30-45°C entfernt. Das Produkt wird per ¹H-NMR charakterisiert und der Wassergehalt der Lösung bestimmt (ca. 60%).

### Synthesebeispiel 4:

### Synthese von TRIS[(-CH₂-CH₂-O)₁₁-SO₃Y]₃ (Y = Na⁺, NH₄⁺) durch Ethoxylierung von TRIS mit 33 EO-Einheiten und anschließender vollständiger Sulfatierung

Das Produkt aus Synthesebeispiel 2 (30 g), Aminosulfonsäure (CAS 5329-14-6; 5.0 g) und katalytische Mengen Harnstoff (17 mg) werden für 7 h auf 85°C erhitzt. Nach Abkühlen auf Raumtemperatur werden 35 g Wasser zugegeben und der pH-Wert der so erhaltenen Lösung wird mit 50%-iger Natronlauge auf pH = 9 eingestellt. Das Produkt wird mittels ¹H-NMR charakterisiert und der Wassergehalt der Lösung bestimmt (ca. 47%).

### Synthesebeispiel 5:

### Synthese von TRIS[(CH₂CH(CH₃)-O)₃-(-CH₂-CH₂-O)₁₃H]₃ durch Propoxylierung von TRIS mit 9 PO-Einheiten und anschließender Ethoxylierung mit 39 EO-Einheiten

In einem Autoklaven wird TRIS (27.7 g) in Toluol (49 g) suspendiert und mit Kalium-*tert*-butylat (0.92 g) versetzt. Es wird dreimal mit N₂ gespült, ein Vordruck von ca. 1.3 bar N₂ eingestellt und die Temperatur auf 135°C erhöht. Propylenoxid (15,3 g) wird so zudosiert, dass die Temperatur bei 135°C bleibt. Anschließend wird 16 h bei 135°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Man erhält 85 g Zwischenprodukt. In einem zweiten Schritt werden 34 g des so erhaltenen Zwischenproduktes in einem Autoklaven in Toluol (70 ml) gelöst und mit Kalium-*tert*-butylat (1.0 g) versetzt. Es wird dreimal mit N₂ gespült, ein Vordruck von ca. 1.3 bar N₂ eingestellt und die Temperatur auf 130°C erhöht. Ethylenoxid (60 g) wird so zudosiert, dass die Temperatur bei 130°C bleibt. Anschließend wird 16 h bei 80°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Auf diese Weise erhält man 73 g der Zielverbindung. OH-Zahl: 67,6 mg/g. Das Produkt wird mittels ¹H-NMR charakterisiert.

### Synthesebeispiel 6:

### Alkoxylierung von TRIS mit 24 Glycidol-Einheiten und 9 Ethylenoxid-Einheiten

In einem Autoklaven wird TRIS (50 g) in Toluol (100 g) suspendiert und mit Kalium-*tert-*butylat (1.7 g) sowie Kronenether [18]Krone-6 (4 g) versetzt. Es wird dreimal mit N₂ gespült, ein Vordruck von ca. 1.3 bar N₂ eingestellt und die Temperatur auf 120°C erhöht. Eine Mischung von Glycidol (142 g) und Ethylenoxid (31.5 g) wird über einen Zeitraum von 8 h so zudosiert, dass die Temperatur bei 120°C bleibt. Anschließend wird 16 h bei 80°C nachgerührt, mit N₂ gespült, auf 70°C abgekühlt und der Reaktor entleert. Das basische Rohprodukt wird mit Hilfe von handelsüblichen Mg-Silikaten neutralisiert, welche anschließend abfiltriert werden. Man erhält 200 g des Zielproduktes. Das Produkt wird mittels ¹H-NMR charakterisiert.

### II) Physikalisch-chemische Untersuchungen

### IIa) Angewandte Methoden:

### Rheologie:

In oszillatorischen Scherexperimenten lassen sich die rheologischen Eigenschaften einer Flüssigkeit bestimmen. Der Speichermodul G' charakterisiert die elastischen Eigenschaften eines Materials, G" die viskosen Eigenschaften.

### Grenzflächenaktivität:

Mittels Du Nouy-Ringmethode (DIN EN 14210) wurde mit einem Tensiometer die Oberflächenspannung des Tensids in wässriger Lösung bestimmt. Aus dem Verlauf der Oberflächenspannung als Funktion der Tensidkonzentration wurde die kritische Mizellbildungskonzentration (cmc) bestimmt.

### Nach der Methode des hängenden Tropfens wurde die Grenzflächenspannung einer wässrigen Tensidlösung zu Hexadekan als Ölphase bestimmt.

Die Methode des maximalen Blasendrucks lieferte Informationen zur Dynamik des Moleküls und zur Stabilität der Mizellen.

### Rheologische Untersuchungen

### Untersuchungsserie 1:

### Untersuchung von TRIS[(-CH₂-CH₂-O)₁₁H]₃

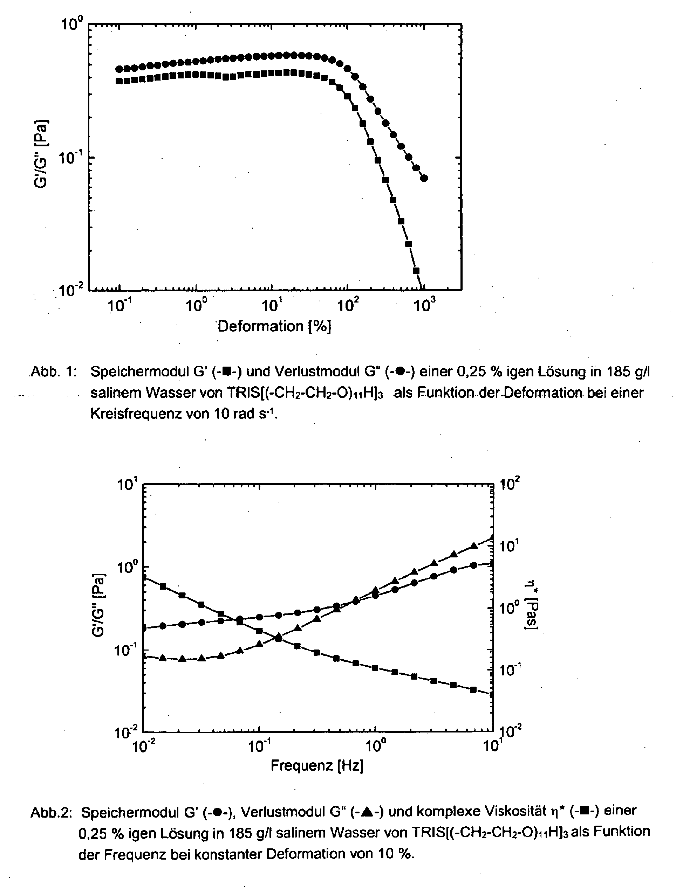

Es wurden die rheologischen Eigenschaften einer 0,25 % igen wässrigen Lösung von TRIS[(-CH₂-CH₂-O)₁₁H]₃ (gemäß Synthesebeispiel 2) bei 25 °C bestimmt. Die Ergebnisse sind in den Abbildungen 1 und 2 zusammengefasst. Diese charakterisieren die viskoelastischen Eigenschaften der Probe (Speicher- und Verlustmodulus) zum einen als Funktion der Deformation und zum Anderen als Funktion der Frequenz einer oszillatorischen Deformation.

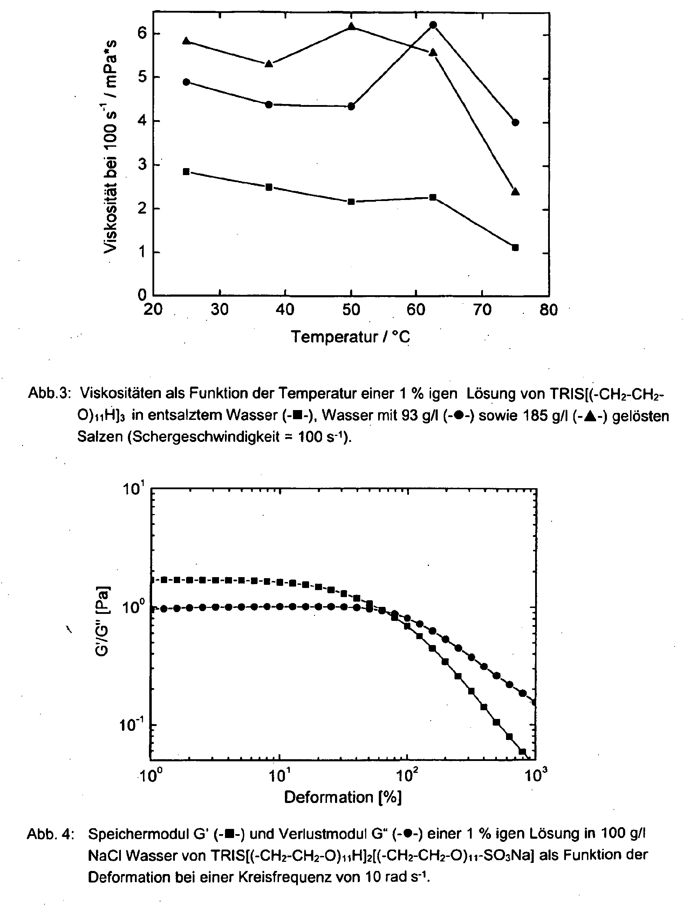

Weiterhin wurde der Einfluss von Elektrolytkonzentration und Temperatur auf die Viskosität einer wässrigen Lösung von TRIS[(-CH₂-CH₂-O)₁₁H]₃ bei einer konstanten Schergeschwindigkeit von 100 s-1 bestimmt. Hierzu wurde jeweils eine 1 % Lösung von TRIS[(-CH₂-CH₂-O)₁₁H]₃ in Wasser ohne Salzzusatz, in Lösung von 93 g/l NaCl und in Lösung von 185 g/l NaCl bei verschiedenen Temperaturen untersucht. Die Ergebnisse sind in Abbildung 3 zusammengefasst und zeigen, dass die Viskosität des untersuchten Moleküls robust gegenüber salzhaltigem Wasser und Temperatur ist.
Grenzflächenaktivität von TRIS[(-CH₂-CH₂-O)₁₁H]₃

### Die Oberflächenspannung beträgt 32 mN/m (1 g/l, 25°C).

### Die kritische Mizellbildungskonzentration in vollentsalztem Wasser ist 0,03 g/l (T = 25°C)

Die Grenzflächenspannung zwischen vollentsalztem Wasser und Hexadekan ist 4,7 mN/m (c = 1 g/l; t = 10 min, T = 25°C)

### Untersuchungen zur Dynamik:

Die Messung der dynamischen Oberflächenspannung zeigt, dass TRIS[(-CH₂-CH₂-O)₁₁H]₃ im Vergleich zu kommerziellen Tensiden wie Alkoholethoxylaten mit Alkylkettenlängen zwischen 10 und 15 C-Atomen und Ethoxylierungsgraden von 5 bis 15 EO kaum dynamisch ist. Das heißt genauer, dass merkliche Grenzflächenaktivität erst nach 2 Sekunden einsetzt, woraus geschlossen werden kann, dass die Mizellen sehr stabil sind. Dies wiederum ist ein Merkmal für viskoelastische Tenside.

### Untersuchungsserie 2:

### Untersuchung von TRIS[(-CH₂-CH₂-O)₁₁H]₂[(-CH₂-CH₂-O)₁₁-SO₃Na]

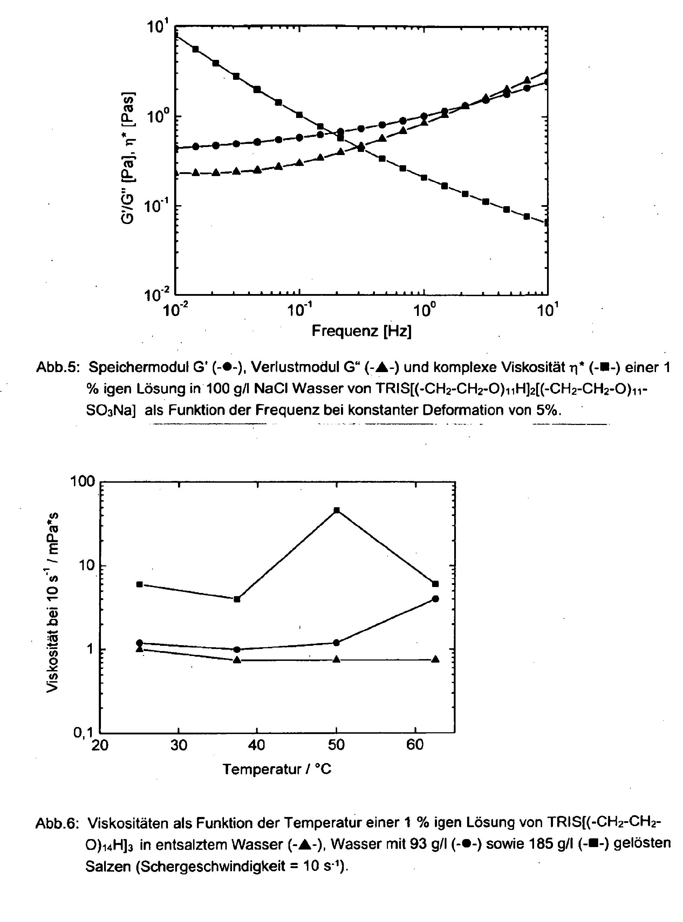

Die Ergebnisse der rheologischen Untersuchungen an TRIS[(-CH₂-CH₂-O)₁₁H]₂[(-CH₂-CH₂-O)₁₁-SO₃Na] sind in den Abbildungen 4 und 5 zusammengefasst. Diese charakterisieren die viskoelastischen Eigenschaften der Probe (Speicher- und Verlustmodulus) zum einen als Funktion der Deformation und zum Anderen als Funktion der Frequenz einer oszillatorischen Deformation.

### Grenzflächenaktivität von TRIS[(-CH₂-CH₂-O)₁₁H]₂[(-CH₂-CH₂-O)₁₁-SO₃Na]

Die Oberflächenspannung in voll entsalztem Wasser beträgt 32,3 mN/m (1 g/l, 25°C). Die Grenzflächenspannung zwischen voll entsalztem Wasser und Hexadekan ist 6,9 mN/m (c = 1 g/l; t = 10 min, T = 25°C). Aus der geringen Dynamik kann auf stabile Mizellen geschlossen werden.

### Untersuchungsserie 3:

### Untersuchung von TRIS[(-CH₂-CH₂-O)₁₄H]₃

Die Ergebnisse der rheologischen Untersuchungen an TRIS[(-CH₂-CH₂-O)₁₄H]₃ (synthetisiert analog Synthesebeispiel 2, nur mit 42 anstelle von 33 EO-Einheiten) zeigt Abbildung 6. Es zeigt sich, dass die viskosen Eigenschaften der Probe durch die Einwirkung von Salz und Temperatur teilweise verstärkt werden.
Grenzflächenaktivität von TRIS[(-CH₂-CH₂-O)₁₄H]₃

Die Oberflächenspannung in voll entsalztem Wasser beträgt 34,8 mN/m (1 g/l, 25°C).

Die Grenzflächenspannung zwischen voll entsalztem Wasser und Hexadekan ist 6,4 mN/m (t = 10 min, T = 25°C). Aus der geringen Dynamik (Messung der dynamischen Oberflächenspannung) kann auf stabile Mizellen geschlossen werden. Untersuchungsserie 4:
Untersuchung des Substitutionsmusters am Aromaten

Es wurden die Oberflächenspannung und die Grenzflächenspannung gegen Hexadekan von 0,1 Gew. %-igen wässrigen Lösungen von erfindungsgemäßen Verbindungen mit verschiedenen Substitutionsmustern an den Phenylringen untersucht, und zwar von den folgenden Verbindungen:
A: TRIS[(-CH₂-CH₂-O)₁₁H]₃
B: TRIS[(-CH₂-CH₂-O)₁₄H]₃
C TRIS[(-CH₂-CH₂-O)₁₁H]₂[(-CH₂-CH₂-O)₁₁-SO₃Na]

Die verschiedenen Substituenten R³ und R⁴ sowie die Ergebnisse sind in Tabelle 4 zusammengestellt. R¹ ist immer H.

**Tabelle 4:**

| Typ | R³ | R⁴ | Oberflächenspannung [mN/m] | | Grenzflächenspannung Hexadekan /Wasser [mN/m] |
|---|---|---|---|---|---|
| | | | Du Nouy Ring | Dynamik (1 s) | |
| A | H | H | 57 | 62 | 19,6 |
| A | Methyl | Methyl | 48 | 62 | 12,8 |
| A | t-Butyl | t-Buty | 33 | 60 | 5,4 |
| B | t-Butyl | t-Butyl | 35 | 58 | 6,4 |
| C | t-Butyl | t-Butyl | 32 | 68 | 6,9 |

Die Ergebnisse zeigen, dass das Substitutionsmuster an den Phenylringen großen Einfluß auf die Tensideigenschaften und auf die Viskoelastizität der erfindungsgemäßen Verbindungen hat.

Das am Aromaten unsubstituierte Molekül hat nur äußerst geringe Grenzflächenaktivität. Die mit Methylgruppen substituierte Verbindung hat geringe Grenzflächenaktivität, während das mit t-Butylgruppen substituierte Produkt eine hohe Grenzflächenaktivität aufweist.

Die Grenzflächenaktivität lässt sich bei den erfindungsgemäßen Verbindungen also auf einfach Art und Weise einstellen.

## Patentansprüche

1. Derivate von Tris(2-hydroxyphenyl)methan, **dadurch gekennzeichnet, dass** die Tris(2-hydroxyphenyl)methan-Derivate die allgemeine Formel (I) 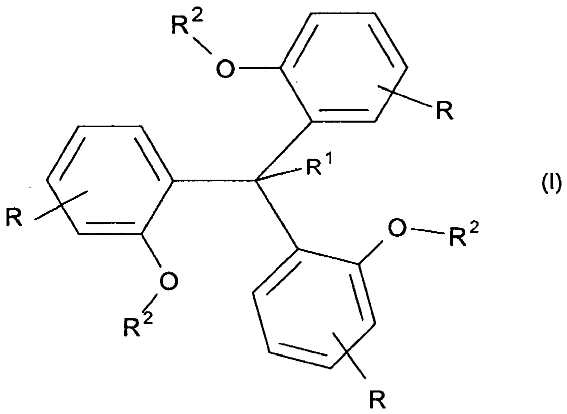 aufweisen, wobei die Reste R¹, R² und R die nachfolgende Bedeutung haben:
R: unabhängig voneinander 0 bis 4 C₁- bis C₃₀-Kohlenwasserstoffreste pro Phenylring,
R¹: ein Rest ausgewählt aus der Gruppe von H, OH, F, Cl, Br, I sowie C₁- bis C₃₀-Kohlenwasserstoffgruppen,
R²: unabhängig voneinander Reste der allgemeinen Formel -(R⁵-O-)ₙ-R⁶-X (III), wobei n für eine Zahl von 1 bis 50 steht, und wobei die Reste R⁵ unabhängig voneinander ausgewählt werden aus der Gruppe von Resten R⁷, R⁸ und R⁹ 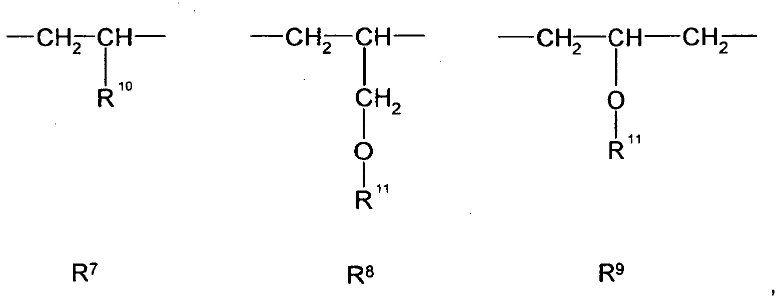 wobei R⁶, X, R¹⁰ und R¹¹ unabhängig voneinander die folgende Bedeutung haben:
R⁶: eine Einfachbindung oder eine Alkylengruppe mit 1 bis 10 Kohlenstoffatomen, welche optional funktionelle Gruppen als Substituenten aufweisen kann,
X : H oder eine hydrophile Gruppe, wobei die hydrophile Gruppe eine saure Gruppe oder ein Rest ist, welcher mindestens eine OH-Gruppe umfasst,
R¹⁰: H oder ein C₁- bis C₆-Kohlenwasserstoffrest,
R¹¹: eine Gruppe -(R⁵-O-)ₘ-R⁶-X, wobei m für eine Zahl von 0 bis 50 steht,
und wobei die Gesamtzahl z aller Gruppen R⁵ in einem Rest R² 1 bis 50 beträgt, mit der Maßgabe, dass es sich bei z um eine Zahl von 2 bis 50 handelt, falls mindestens ein X für H steht.

2. Verbindungen gemäß Anspruch 1, **dadurch gekennzeichnet, dass** die Verbindungen die allgemeine Formel (II) 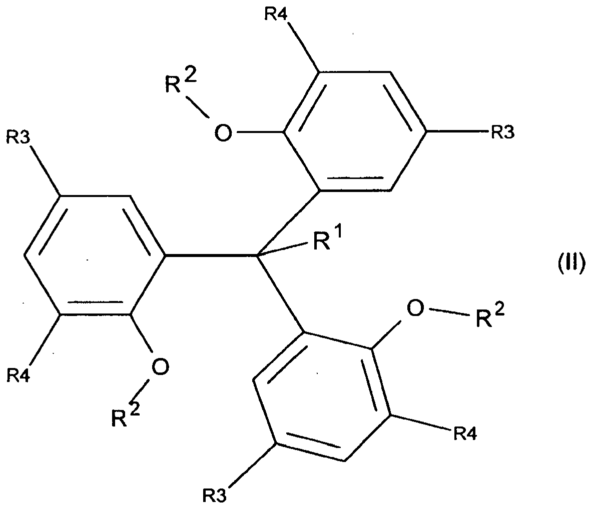 aufweisen, wobei R¹ und R² die oben dargestellte Bedeutung haben und R³ und R⁴ unabhängig voneinander für H oder einen C₁- bis C₃₀-Kohlenwasserstoffrest stehen.

3. Verbindungen gemäß Anspruch 3, **dadurch gekennzeichnet dass** es sich bei R³ und R⁴ unabhängig voneinander um geradkettige oder verzweigte aliphatische C₁- bis C₆- Kohlenwasserstoffreste handelt.

4. Verbindungen gemäß Anspruch 3, **dadurch gekennzeichnet, dass** es sich bei R³ und R⁴ jeweils um t-Butylreste handelt.

5. Verbindungen gemäß einem der Ansprüche 1 bis 4, **dadurch gekennzeichnet, dass** z für eine Zahl von 5 bis 30 steht.

6. Verbindungen gemäß einem der Ansprüche 1 bis 5 **dadurch gekennzeichnet, dass** es sich bei X um eine saure Gruppe ausgewählt aus der Gruppe von Carboxylgruppen -COOM, Sulfonsäuregruppen -SO₃M, Sulfatgruppen -OSO₃M, Phosphonsäuregruppen -PO₂M₂ oder Phosphorsäuregruppen -OPO₃M₂ handelt, wobei M für H⁺ oder ein k-wertiges Gegenion ¹/ₖY^{k+} steht.

7. Verbindungen gemäß einem der Ansprüche 1 bis 5 **dadurch gekennzeichnet, dass** es sich bei X um einen Rest, welcher mindestens eine OH-Gruppe umfasst, ausgewählt aus der Gruppe bestehend aus Mono- und Oligosaccharidresten handelt.

8. Verbindungen gemäß einem der Ansprüche 1 bis 7, **dadurch gekennzeichnet, dass** es sich bei mindestens einem der Reste R⁵ um einen Rest R⁷ handelt, wobei die Reste R¹⁰ in R⁷ unabhängig voneinander für H, Methyl oder Ethyl stehen.

9. Verbindungen gemäß Anspruch 8, **dadurch gekennzeichnet, dass** es sich bei mindestens 50 % der Reste R¹⁰ um H handelt.

10. Verbindungen gemäß einem der Ansprüche 1 bis 4, **dadurch gekennzeichnet, dass** es sich bei den Resten R² unabhängig voneinander um Reste der allgemeinen Formel -(R⁷-O-)ₙ-R⁶-X (IIIa) handelt, wobei die Reste R¹⁰ in R⁷ unabhängig voneinander aus der Gruppe von H, Methyl oder Ethyl gewählt werden, mit der Maßgabe, dass es sich bei mindestens 50 % der Reste R¹⁰ um H handelt und wobei n für eine Zahl von 5 bis 30 steht.

11. Verbindung gemäß einem der Ansprüche 1 bis 4, **dadurch gekennzeichnet, dass** es sich bei den Resten R² unabhängig voneinander um Reste der allgemeinen Formel -(R⁷-O-)ₙ-H (IIIb) handelt, wobei die Reste R¹⁰ in R⁷ unabhängig voneinander aus der Gruppe von H, Methyl oder Ethyl gewählt werden, mit der Maßgabe, dass es sich bei mindestens 50 % der Reste R¹⁰ um H handelt und wobei n für eine Zahl von 5 bis 30 steht.

12. Verbindungen gemäß einem der Ansprüche 1 bis 4, **dadurch gekennzeichnet, dass** es sich bei den Resten R² unabhängig voneinander um Reste der allgemeinen Formel -(-CH₂-CH(R¹²)-O-)ₐ-(-CH₂-CH₂-O-)_{b}-H (IIIc) handelt, wobei die Alkylenoxid-Blöcke in dieser Reihenfolge angeordnet sind, es sich bei R¹² um Kohlenwasserstoffreste mit 1 bis 6 Kohlenstoffatomen handelt und a und b jeweils für Zahlen von 1 bis 49 stehen, mit der Maßgabe, dass die Summe aus a und b 2 bis 50 beträgt.

13. Verbindungen gemäß einem der Ansprüche 1 bis 4, **dadurch gekennzeichnet, dass** es sich bei den Resten R² unabhängig voneinander um Reste -(-CH₂-CH(R¹³)-O-)ₙ-R⁶-X (IIId) handelt, wobei bei mindestens einem der Reste R² X nicht für H steht und R¹³ aus der Gruppe von H, Methyl und Ethyl ausgewählt wird, mit der Maßgabe, dass es sich bei mindestens 20 % der Reste R⁵ um H handelt.

14. Verbindung gemäß Anspruch 13, **dadurch gekennzeichnet, dass** es sich bei der Gruppe X um Carboxylgruppen -COOM, Sulfonsäuregruppen -SO₃M oder Sulfatgruppen -OSO₃M handelt, wobei M wie oben definiert ist.

15. Verbindung gemäß einem der Ansprüche 1 bis 5, **dadurch gekennzeichnet, dass** der Rest der allgemeinen Formel (III) Reste R⁸ und/oder R⁹ umfasst.

16. Derivate von Tris(2-hydroxyphenyl)methan, erhältlich indem man Carbonsäurevinylester in Gegenwart von Verbindungen gemäß einem der Ansprüche 1 bis 15 radikalisch polymerisiert, und die polymerisierten Vinylestergruppen anschließend zumindest teilweise zu OH-Gruppen hydrolysiert.

17. Verfahren zur Herstellung der Verbindungen gemäß einem der Ansprüche 1 bis 15, **dadurch gekennzeichnet, dass** man eine Ausgangsverbindung der allgemeinen Formel (IV) 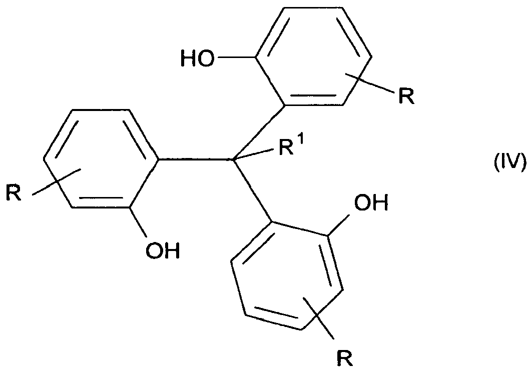 bereitstellt, mit Reagenzien ausgewählt aus der Gruppe von C₂- bis C₈-Alkylenoxiden, Glycidol und cyclischen C₃- bis C₉-Carbonaten alkoxyliert, und hierauf optional die terminalen H-Atome zumindest teilweise durch Reste -R⁶-X substituiert.

18. Verwendung von Verbindungen gemäß einem der Ansprüche 1 bis 16 als Tenside und Verdicker.

19. Verwendung von Verbindungen gemäß einem der Ansprüche 1 bis 16 zur Herstellung von Wasch- und Reinigungsmitteln, Farben und Anstrichmittel, kosmetischen und pharmazeutischen Formulierungen, Papier-, Textil- und Lederhilfsmitteln, Formulierungen für die Human- und Tierernährung, den Bausektor, Pflanzenschutzformulierungen sowie allgemein zur Herstellung von Emulsionen und Dispersionen.

## Claims

1. A derivative of tris(2-hydroxyphenyl)methane, wherein the tris(2-hydroxyphenyl)methane derivative has the general formula (I) 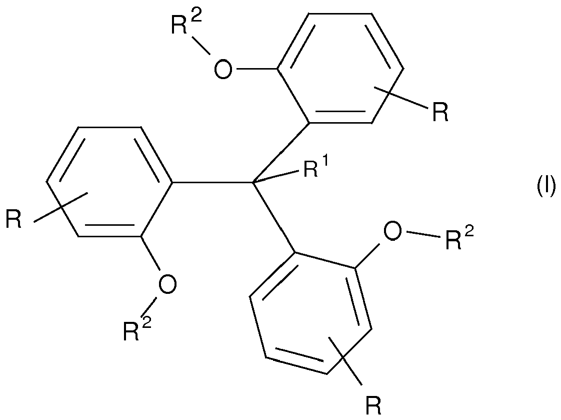 where the radicals R¹, R² and R have the following meaning:
R: independently of one another, 0 to 4 C₁- to C₃₀-hydrocarbon radicals per phenyl ring,
R¹: a radical selected from the group of H, OH, F, Cl, Br, I and C₁- to C₃₀-hydrocarbon groups,
R²: independently of one another, radicals of the general formula -(R⁵-O-)ₙ-R⁶-X (III), where n is a number from 1 to 50, and where the radicals R⁵, independently of one another, are selected from the group of radicals R⁷, R⁸ and R⁹ 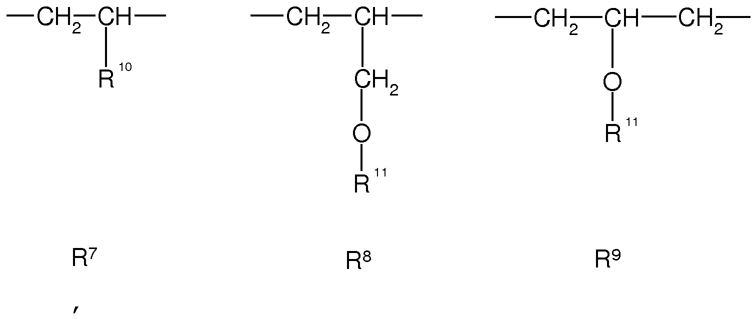 where R⁶, X, R¹⁰ and R¹¹, independently of one another, have the following meaning:
R⁶ : a single bond or an alkylene group having 1 to 10 carbon atoms which can have optionally functional groups as substituents,
X : H or a hydrophilic group, where the hydrophilic group is an acidic group or a radical which comprises at least one OH group,
R¹⁰: H or a C₁- to C₆-hydrocarbon radical,
R¹¹: a group -(R⁵-O-)ₘ-R⁶-X, where m is a number from 0 to 50,
and where the total number z of all groups R⁵ in a radical R² is 1 to 50, with the proviso that z is a number from 2 to 50, if at least one X is H.

2. The compound according to claim 1, wherein the compound has the general formula (II) 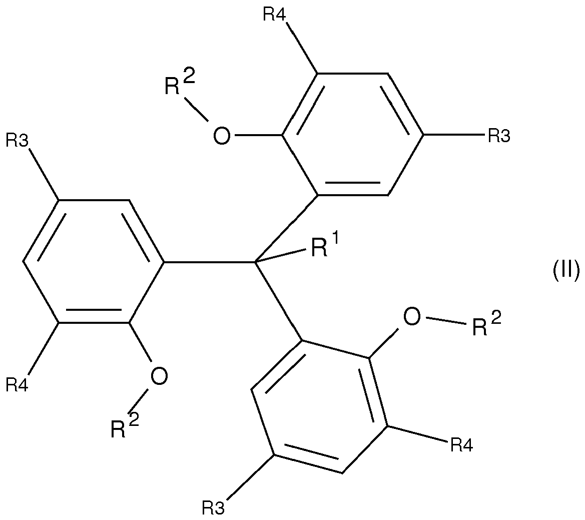 where R¹ and R² have the meaning given above and R³ and R⁴, independently of one another, are H or a C₁- to C₃₀-hydrocarbon radical.

3. The compound according to claim 3, wherein R³ and R⁴, independently of one another, are straight-chain or branched aliphatic C₁- to C₆-hydrocarbon radicals.

4. The compound according to claim 3, wherein R³ and R⁴ are in each case t-butyl radicals.

5. The compound according to any one of claims 1 to 4, wherein z is a number from 5 to 30.

6. The compound according to any one of claims 1 to 5, wherein X is an acidic group selected from the group of carboxyl groups -COOM, sulfonic acid groups -SO₃M, sulfate groups -OSO₃M, phosphonic acid groups -PO₂M₂ or phosphoric acid groups-OPO₃M₂, where M is H⁺ or a k-valent counterion ¹/ₖY^{k+}.

7. The compound according to any one of claims 1 to 5, wherein X is a radical which comprises at least one OH group selected from the group consisting of mono- and oligosaccharide radicals.

8. The compound according to any one of claims 1 to 7, wherein at least one of the radicals R⁵ is a radical R⁷, where the radicals R¹⁰ in R⁷, independently of one another, are H, methyl or ethyl.

9. The compound according to claim 8, wherein at least 50% of the radicals R¹⁰ are H.

10. The compound according to any one of claims 1 to 4, wherein the radicals R², independently of one another, are radicals of the general formula -(R⁷-O-)ₙ-R⁶-X (IIIa), where the radicals R¹⁰ in R⁷, independently of one another, are selected from the group of H, methyl or ethyl, with the proviso that at least 50% of the radicals R¹⁰ are H and where n is a number from 5 to 30.

11. The compound according to any one of claims 1 to 4, wherein the radicals R², independently of one another, are radicals of the general formula -(R⁷-O-)ₙ-H (IIIb), where the radicals R¹⁰ in R⁷, independently of one another, are selected from the group of H, methyl or ethyl, with the proviso that at least 50% of the radicals R¹⁰ are H and where n is a number from 5 to 30.

12. The compound according to any one of claims 1 to 4, wherein the radicals R², independently of one another, are radicals of the general formula -(--CH₂-CH(R¹²)-O-)ₐ-(--CH₂-CH₂-O-)_{b}-H (IIIc), where the alkylene oxide blocks are arranged in this order, R¹² is hydrocarbon radicals having 1 to 6 carbon atoms and a and b are in each case numbers from 1 to 49, with the proviso that the sum of a and b is 2 to 50.

13. The compound according to any one of claims 1 to 4, wherein the radicals R², independently of one another, are radicals -(--CH₂-CH(R¹³)-O-)ₙ-R⁶-X (IIId), where in at least one of the radicals R² X is not H and R¹³ is selected from the group of H, methyl and ethyl, with the proviso that at least 20% of the radicals R⁵ are H.

14. The compound according to claim 13, wherein the group X is carboxyl groups -COOM, sulfonic acid groups -SO₃M or sulfate groups -OSO₃M, where M is as defined above.

15. The compound according to any one of claims 1 to 5, wherein the radical of the general formula (III) comprises radicals R⁸ and/or R⁹.

16. A derivative of tris(2-hydroxyphenyl)methane, obtainable by free-radically polymerizing carboxylic acid vinyl esters in the presence of compounds according to any one of claims 1 to 15, and then hydrolyzing the polymerized vinyl ester groups at least partially to give OH groups.

17. A process for the preparation of the compounds according to any one of claims 1 to 15, wherein a starting compound of the general formula (IV) 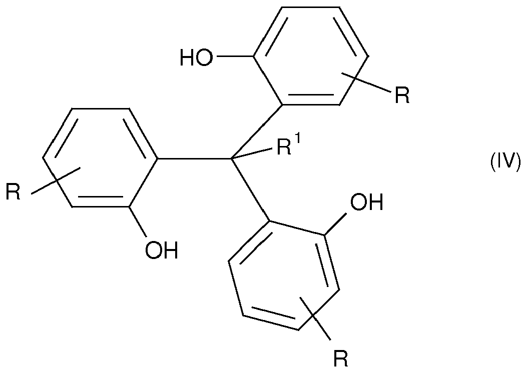 is prepared, alkoxylated with reagents selected from the group of C₂- to C₈-alkylene oxides, glycidol and cyclic C₃- to C₉-carbonates, and then optionally the terminal H atoms are substituted at least partially by radicals -R⁶-X.

18. The use of compounds according to any one of claims 1 to 16 as surfactants and thickeners.

19. The use of compounds according to any one of claims 1 to 16 for producing detergents and cleaners, paints and coatings, cosmetic and pharmaceutical formulations, paper, textile and leather auxiliaries, formulations for human and animal nutrition, the construction sector, crop protection formulations, and generally for producing emulsions and dispersions.

## Revendications

1. Dérivés de tris(2-hydroxyphényl)méthane, **caractérisés en ce que** les dérivés de tris(2-hydroxyphényl)méthane présentent la formule générale (I) 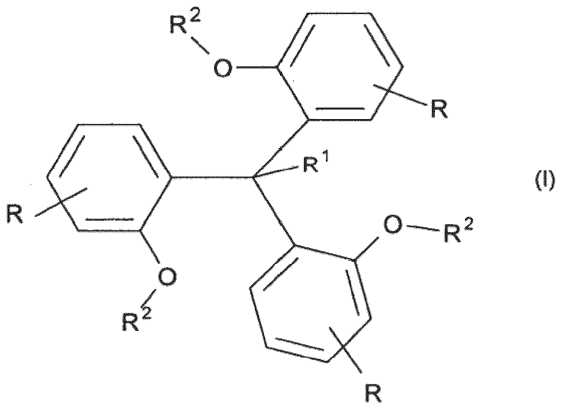 dans laquelle les radicaux R¹, R² et R ont la signification suivante :
R : indépendamment les uns des autres 0 à 4 radicaux hydrocarbonés en C₁ à C₃₀ par cycle phényle,
R¹ : un radical choisi dans le groupe constitué par H, OH, F, Cl, Br, I, ainsi que les groupes hydrocarbonés en C₁ à C₃₀,
R² : indépendamment les uns des autres des radicaux de formule générale -(R⁵-O-)ₙ-R⁶-X (III), dans laquelle n représente un nombre de 1 à 50 et les radicaux R⁵ sont choisis indépendamment les uns des autres dans le
groupe constitué par les radicaux R⁷, R⁸ et R⁹, 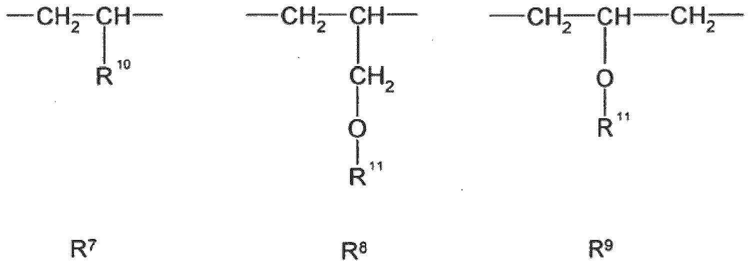
R⁶, X, R¹⁰ et R¹¹ ayant indépendamment les uns des autres la signification suivante :
R⁶ : une simple liaison ou un groupe alkylène de 1 à 10 atomes de carbone, qui peut éventuellement comprendre des groupes fonctionnels en tant que substituants,
X : H ou un groupe hydrophile, le groupe hydrophile étant un groupe acide ou un radical qui comprend au moins un groupe OH,
R¹⁰ : H ou un radical hydrocarboné en C₁ à C₆,
R¹¹ : un groupe -(R⁵-O-)ₘ-R⁶-X, m représentant un nombre de 0 à 50,
et le nombre total z de tous les groupes R⁵ dans un radical R² étant de 1 à 50, à condition que z soit un nombre de 2 à 50 si au moins un X représente H.

2. Composés selon la revendication 1, **caractérisés en ce que** les composés présentent la formule générale (II) 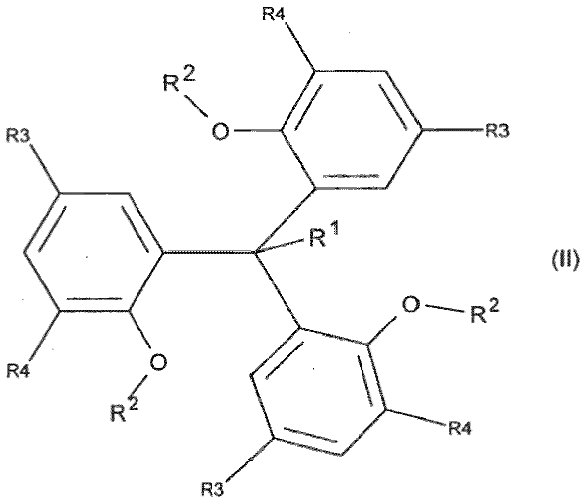 dans laquelle R¹ et R² ont la signification indiquée précédemment et R³ et R⁴ représentent indépendamment l'un de l'autre H ou un radical hydrocarboné en C₁ à C₃₀.

3. Composés selon la revendication 3, **caractérisés en ce que** R³ et R⁴ sont indépendamment l'un de l'autre des radicaux hydrocarbonés en C₁ à C₆ aliphatiques linéaires ou ramifiés.

4. Composés selon la revendication 3, **caractérisés en ce que** R³ et R⁴ sont chacun des radicaux t-butyle.

5. Composés selon l'une quelconque des revendications 1 à 4, **caractérisés en ce que** z représente un nombre de 5 à 30.

6. Composés selon l'une quelconque des revendications 1 à 5, **caractérisés en ce que** X est un groupe acide choisi dans le groupe constitué par les groupes carboxyle -COOM, les groupes acide sulfonique - SO₃M, les groupes sulfate -OSO₃M, les groupes acide phosphonique -PO₂M₂ ou les groupes acide phosphorique - OPO₃M₂, M représentant H⁺ ou un contre-ion k-valent ¹/ₖY^{k+}.

7. Composés selon l'une quelconque des revendications 1 à 5, **caractérisés en ce que** X est un radical qui comprend au moins un groupe OH, choisi dans le groupe constitué par les radicaux mono- et oligosaccharides.

8. Composés selon l'une quelconque des revendications 1 à 7, **caractérisés en ce qu'**au moins un des radicaux R⁵ est un radical R⁷, les radicaux R¹⁰ dans R⁷ représentant indépendamment les uns des autres H, méthyle ou éthyle.

9. Composés selon la revendication 8, **caractérisés en ce qu'**au moins 50 % des radicaux R¹⁰ représentent H.

10. Composés selon l'une quelconque des revendications 1 à 4, **caractérisés en ce que** les radicaux R² représentent indépendamment les uns des autres des radicaux de formule générale -(R⁷-O-)ₙ-R⁶-X (IIIa), les radicaux R¹⁰ dans R⁷ étant choisis indépendamment les uns des autres dans le groupe constitué par H, méthyle ou éthyle, à condition qu'au moins 50 % des radicaux R¹⁰ représentent H, et n représentant un nombre de 5 à 30.

11. Composés selon l'une quelconque des revendications 1 à 4, **caractérisés en ce que** les radicaux R² représentent indépendamment les uns des autres des radicaux de formule générale -(R⁷-O-)ₙ-H (IIIb), les radicaux R¹⁰ dans R⁷ étant choisis indépendamment les uns des autres dans le groupe constitué par H, méthyle ou éthyle, à condition qu'au moins 50 % des radicaux R¹⁰ représentent H, et n représentant un nombre de 5 à 30.

12. Composés selon l'une quelconque des revendications 1 à 4, **caractérisés en ce que** les radicaux R² représentent indépendamment les uns des autres des radicaux de formule générale -(-CH₂-CH(R¹²)-O-)ₐ-(-CH₂-CH₂-O-)_{b}-H (IIIc), les séquences oxyde d'alkylène étant agencées dans cet ordre, R¹² représentant des radicaux hydrocarbonés de 1 à 6 atomes de carbone, et a et b représentant chacun des nombres de 1 à 49, à condition que la somme de a et b soit de 2 à 50.

13. Composés selon l'une quelconque des revendications 1 à 4, **caractérisés en ce que** les radicaux R² représentent indépendamment les uns des autres des radicaux -(-CH₂-CH(R¹³)-O-)ₙ-R⁶-X (IIId), X ne représentant pas H dans au moins un des radicaux R² et R¹³ étant choisi dans le groupe constitué par H, méthyle et éthyle, à condition qu'au moins 20 % des radicaux R⁵ représentent H.

14. Composé selon la revendication 13, **caractérisé en ce que** le groupe X représente des groupes carboxyle-COOM, des groupes acide sulfonique -SO₃M ou des groupes sulfate -OSO₃M, M étant tel que défini précédemment.

15. Composé selon l'une quelconque des revendications 1 à 5, **caractérisé en ce que** le radical de formule générale (III) comprend des radicaux R⁸ et/ou R⁹.

16. Dérivés de tris(2-hydroxyphényl)méthane, pouvant être obtenus par polymérisation radicalaire d'esters vinyliques d'acides carboxyliques en présence de composés selon l'une quelconque des revendications 1 à 15, puis hydrolyse au moins partielle des groupes ester vinylique polymérisés en groupes OH.

17. Procédé de fabrication des composés selon l'une quelconque des revendications 1 à 15, **caractérisé en ce qu'**un composé de départ de formule générale (IV) 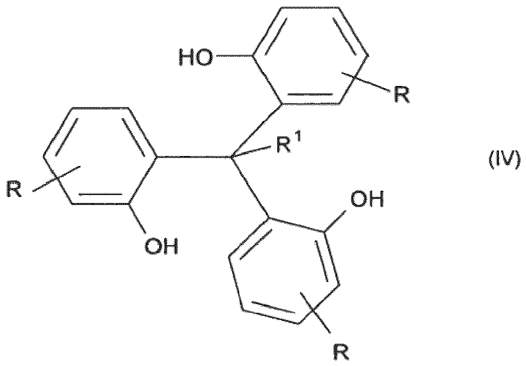 est préparé, alcoxylé avec des réactifs choisis dans le groupe constitué par les oxydes d'alkylène en C₂ à C₈, le glycidol et les carbonates cycliques en C₃ à C₉, et les atomes H terminaux sont éventuellement au moins partiellement remplacés par des radicaux -R⁶-X.

18. Utilisation de composés selon l'une quelconque des revendications 1 à 16 en tant que tensioactifs et épaississants.

19. Utilisation de composés selon l'une quelconque des revendications 1 à 16 pour la fabrication de détergents, d'encres et de peintures, de formulations cosmétiques et pharmaceutiques, d'adjuvants pour papier, textile et cuir, de formulations pour l'alimentation humaine et animale, le secteur du bâtiment, de formulations pour la protection des plantes, ainsi que de manière générale pour la fabrication d'émulsions et de dispersions.
